# EUROPÄISCHE PATENTSCHRIFT

(11) **EP 3 528 728 B1**
(45) Veröffentlichungstag und Bekanntmachung des Hinweises auf die Patenterteilung: **06.09.2023**
(21) Anmeldenummer: 17783429.8
(22) Anmeldetag: 03.10.2017
(51) Int. Cl.: A61B 17/88, A61F 2/46, A61M 39/10, A61M 5/31, A61C 5/62

(54) **MODULARE AUSTRAGVORRICHTUNG MIT TRENNELEMENT**
MODULAR DISCHARGE DEVICE WITH SEPARATOR ELEMENT
DISPOSITIF DE DISTRIBUTION MODULAIRE AVEC ÉLÉMENT DE SÉPARATION

(30) Priorität: 24.10.2016 CH 14172016; 13.03.2017 CH 2982017
(43) Veröffentlichungstag der Anmeldung: 28.08.2019
(73) Patentinhaber: medmix Switzerland AG, 9469 Haag (Rheintal) (CH)
(72) Erfinder: NIEBER, Benjamin, 6274 Eschenbach (CH); VEID, Martin, 6353 Weggis (CH); MATHYS, Beat, 5630 Muri (CH); KUGLER, Stefan, 8047 Zürich (CH)
(74) Vertreter: Manitz Finsterwald Patent- und Rechtsanwaltspartnerschaft mbB
(86) Internationale Anmeldenummer: PCT/EP2017/075061
(87) Internationale Veröffentlichungsnummer: WO 2018/077575

(56) Entgegenhaltungen:
- EP-A1- 2 436 342
- EP-A1- 3 042 679
- DE-A1- 10 247 963
- US-A1- 2004 087 906
- US-A1- 2006 264 964
- US-A1- 2015 045 768

## Beschreibung

### TECHNISCHES GEBIET

Die vorliegende Erfindung betrifft Trennelemente, welche dazu ausgebildet sind, den Durchtritt eines Granulats zu verhindern, aber den Durchtritt von Flüssigkeit zu erlauben, Austragvorrichtungen, die spezifisch dafür ausgestaltet sind, mit solchen Trennelementen eingesetzt zu werden. Ein Verfahren zur Handhabung solcher Austragvorrichtungen wird ebenfalls beschrieben, das keinen Teil der beanspruchten Erfindung bildet. Es dient lediglich dem Verständnis der Erfindung.

### STAND DER TECHNIK

In verschiedenen medizinischen Bereichen wie der Zahnheilkunde, der Orthopädie oder der rekonstruktiven Chirurgie kommen Knochenersatzmaterialien (Bone-Graft- Materialien) zum Einsatz. Das Knochenersatzmaterial besteht meist aus einem Granulat natürlichen oder synthetischen Ursprungs (z.B. ein zweiphasiges Kalziumphosphat/Hydroxylapatit-Material) und einer Flüssigkeit (z.B. körpereigenes Blut oder eine physiologische Natriumchloridlösung).

Herkömmlicherweise wird das Granulat in einer Schale bereitgestellt, und die flüssige Phase wird zugegeben, wodurch das Granulat benetzt wird. Um das so hergestellte fertige Knochenersatzmaterial am Patienten zu applizieren, wird es mit einem Spatel an den Bestimmungsort gebracht. Je nach Lage des Bestimmungsortes kann dies schwierig bis unmöglich sein, und da das Material lose und angehäuft auf dem Spatel liegt, besteht die Gefahr, dass das Material bei der Zuführung vom Spatel rutschen kann. Zudem ist eine solche Handhabung zeitaufwendig. Im Stand der Technik wurde daher vorgeschlagen, das Granulat in einer Spritze aufzunehmen und direkt in der Spritze mit der Flüssigkeit zu benetzen. Um das Granulat in der Spritze zurückzuhalten, aber die Aufnahme der Flüssigkeit in die Spritze zu ermöglichen, ist es bekannt, an der Spritze einen Filter oder ein Sieb vorzusehen.

So ist aus der EP 0 470 393 B eine Granulatspritze bekannt, bei welcher eine Kappe mit einer Scheibe aus porösem Material auf das Austrittsende des Spritzengehäuses aufgesteckt oder aufgeschraubt ist. Um das Gemisch auszutragen, wird die Kappe entfernt, und ein Kolben wird vorgestoßen.

Die EP 1 093 767 B offenbart eine Spritze mit einem Spritzenzylinder, welcher mit Granulat gefüllt ist. Am Zylinder ist eine Kanülenspitze mittels Reibpassung angebracht, welche über ein Sieb in einer Aussparung verfügt. Über eine Öffnung wird Blut in den Spritzenzylinder eingesaugt. Ist das Blut genügend mit dem Granulat vermischt, so wird die Kanülenspitze manuell von dem Spritzenzylinder herunter geschoben.

Aus der EP 2 436 342 B ist eine Spritze zur Applikation von Knochenersatzmaterial bekannt, die einen Zylinder aufweist, in dem Granulat aufgenommen ist. Der Zylinder weist an seinem distalen Ende ein Aussengewinde auf, auf dem ein abnehmbarer Aufsatz mit einem Innengewinde gehalten wird. Der Aufsatz weist eine Einspritzöffnung sowie Drainageöffnungen auf. Flüssigkeit wird durch die Einspritzöffnung in den Zylinder injiziert, um das Granulat zu benetzen und zu quellen, und überschüssige Flüssigkeit wird anschließend durch die Drainageöffnungen wieder ausgepresst. Ist das Knochenersatzmaterial genügend mit Flüssigkeit benetzt, wird der Aufsatz abgeschraubt und durch eine gekrümmte Entladungsdüse ersetzt.

Weitere bekannte Spritzen sind beispielsweise in der DE 102 47 963 A1, EP 3 042 679 A1, US 2004/087906 A1, US 2015/045768 A1 und US 2006/264964 A1 bekannt.

Bei derartigen Spritzen besteht jedoch noch Verbesserungsbedarf hinsichtlich der Handhabung.

### DARSTELLUNG DER ERFINDUNG

Die vorliegende Erfindung wird im Anspruch 1 definiert. Vorteilhafte Weiterbildungen der Erfindung sind Gegenstand der abhängigen Ansprüche.

Die vorliegende Erfindung stellt ein Trennelement für eine Austragvorrichtung zum Austragen eines Produkts zur Verfügung. Das Trennelement definiert ein proximales Ende und ein distales Ende. Es weist einen Filterbereich auf, der dazu ausgebildet ist, den Durchtritt eines Granulats entlang einer axialen Richtung zu verhindern, aber den Durchtritt von Flüssigkeit entlang der axialen Richtung zu erlauben. Das Trennelement weist ausserdem ein zum proximalen Ende hin offenes Innengewinde auf, welches um die axiale Richtung verläuft, und mindestens einen aussenumfänglich angeordneten, federelastischen Rastarm, welcher im Bereich des proximalen Endes des Trennelements ausgebildet ist und sich mit seinem freien Ende in die proximale Richtung zum proximalen Ende hin erstreckt. Zusätzlich weist das Trennelement ein oder mehrere Mitnahmeelemente auf, die außenseitig auf dem Trennelement ausgebildet sind, um in Umfangsrichtung einen formschlüssigen Eingriff des Trennelements mit einem Mitnehmer eines Verschlusses zu ermöglichen. Der Begriff «Gewinde» ist hier breit zu verstehen. Er umfasst jegliche Verbindungsstrukturen, mit deren Hilfe zwei Teile durch eine kombinierte rotatorische und axiale Bewegung zunehmend geführt in Eingriff bringbar sind. Darunter fallen insbesondere auch Strukturen, wie sie zu Herstellung einer Bajonettverbindung benötigt werden. Als «Richtung» des Gewindes wird die Richtung der rotatorischen Komponente der Eingriffsbewegung bezeichnet. Bei den in diesem Dokument angegebenen Gewinden kann es sich insbesondere um Gewinde im engeren, üblichen Sinne handeln, also um ein- oder mehrgängige Gewinde mit einem oder mehreren helikalen Gewindegängen.

Das Trennelement lässt sich mit Hilfe seines Innengewindes auf ein passendes Außengewinde im Bereich einer Austragöffnung eines Behälters aufschrauben. Am Trennelement lässt sich außerdem ein Verschluss anbringen. Dazu kann das Trennelement an seinem distalen Ende ebenfalls eine geeignete Verbindungstruktur, z.B. ein zum distalen Endes hin offenes Außengewinde (im oben definierten, breiten Sinn), aufweisen, welches koaxial zum Innengewinde ebenfalls um die axiale Richtung verläuft.

Um zu verhindern, dass der Verschluss das Trennelement mitnimmt, wenn der Verschluss vom Trennelement entfernt wird, weist das Trennelement den mindestens einen federelastischen Rastarm auf. Der mindestens eine Rastarm dient dazu, einen Rasteingriff mit dem Behälter einzugehen, um das Trennelement am Behälter zu sichern, wenn der Verschluss vom Trennelement entfernt wird. Vorzugsweise ist dieser Rasteingriff erst dann lösbar, nachdem der Verschluss mindestens teilweise vom Trennelement entfernt wurde, d.h. der Rastarm ist vorzugsweise derart angeordnet, dass der Verschluss eine radiale Auslenkung des Rastarms nach außen blockieren kann, wenn er am Trennelement angebracht ist. Bevorzugt sind zwei oder mehr derartige Rastarme vorhanden, die über den Umfang des Trennelements verteilt angeordnet sind, um eine möglichst gleichmäßige Sicherung des Trennelements am Behälter sicherzustellen. Es sei darauf hingewiesen, dass Aussagen, welche im Folgenden für einen einzelnen Rastarm gemacht werden, gleichermaßen für jeden weiteren Rastarm im Falle einer Vielzahl von Rastarmen gelten, und dass Aussagen, die sich auf eine Mehrzahl von Rastarmen beziehen, auch gelten, wenn nur ein einzelner Rastarm vorhanden ist.

Der Filterbereich des Trennelements dient dazu, ein Granulat (z.B. eine Komponente eines Knochenersatzmaterials) im Behälter zurückzuhalten, aber die Aufnahme von Flüssigkeit in den Behälter zu erlauben. Der Filterbereich kann z.B. als Siebplatte mit einer Mehrzahl von axialen Durchlassöffnungen ausgebildet sein. Alternativ kann der Filterbereich auch z.B. aus einem porösen Material gebildet sein, das ein Netzwerk miteinander verbundener Kanäle bildet. Auf diese Weise ermöglicht es das Trennelement, eine Flüssigkeit wie z.B. Blut oder Kochsalzlösung in den Behälter einzusaugen oder einzuspritzen, ohne dass ein im Behälter aufgenommenes Granulat, insbesondere für ein Knochenersatzmaterial, aus dem Behälter austreten kann. Bevorzugt sind der Filterbereich, das Innengewinde und die Federarme sowie ggfs. das Außengewinde einstückig miteinander ausgebildet. In einigen Ausführungsformen erstreckt sich der Filterbereich bis zum distalen Ende des Trennelements. In diesem Fall bildet also das distale Ende des Filterbereichs auch das distale Ende des Trennelements insgesamt. Dies erleichtert es, eine Flüssigkeit aus einer Schale oder einer Vertiefung möglichst vollständig aufzunehmen. Das Außengewinde, sofern vorhanden, kann dabei den Filterbereich umgeben, also auf der radialen Außenseite des Filterbereichs ausgebildet sein. Eine solche Ausführung kann allerdings fertigungstechnisch problematisch sein. In anderen Ausführungsformen ist daher das Außengewinde an einem Rohrstutzen ausgebildet, der sich in distaler Richtung an den Filterbereich anschließt. Der Rohrstutzen definiert dann innenseitig einen Hohlraum, wobei der Hohlraum in proximaler Richtung vom Filterbereich begrenzt ist. Dies ist fertigungstechnisch vorteilhaft, da für die Entformung des Gewindes ein einfacher Drehkern eingesetzt werden kann.

Bevorzugt ist das Trennelement von drehsymmetrischer Grundform bezüglich Drehungen um eine zentrale Längsachse, die entlang der axialen Richtung verläuft. Der mindestens eine Rastarm weist vorzugsweise im Bereich seines freien Endes innenseitig ein Hintergreifelement auf. Dieses dient dazu, eine komplementäre Struktur am Behälter zu hintergreifen, um so das Trennelement am Behälter zu sichern. Das Hintergreifelement kann insbesondere die Form einer sich radial nach innen erstreckenden Rastnase haben.

Alternativ oder zusätzlich kann der mindestens eine Rastarm im Bereich seines freien Endes innenseitig eine Eingriffsstruktur aufweisen, die dazu ausgebildet ist, mit einer Drehsicherungsstruktur am Behälter zusammenzuwirken. Auf diese Weise kann anstelle oder zusätzlich zu einer axialen Sicherung auch eine Drehsicherung erreicht werden. Die Eingriffsstruktur kann insbesondere in Form einer innenseitigen Verzahnung, die um die axiale Richtung verläuft, ausgebildet sein. Um zu erleichtern, dass der Verschluss radiale Auslenkungen des Rastarms nach außen blockieren kann, ist es von Vorteil, wenn der mindestens eine Rastarm derart in einem Bereich des Außenumfangs des Trennelements am Trennelement ausgebildet ist, dass eine Außenfläche des mindestens einen Rastarms zu einer distal vom Rastarm gelegenen Außenfläche des Trennelements bündig oder radial nach außen versetzt angeordnet ist. Insbesondere ist es von Vorteil, wenn der mindestens eine Rastarm den maximalen Radius des Trennelements quer zur axialen Richtung definiert. Dadurch kann die Mantelwand eines Verschlusses ohne Weiteres auf der Außenfläche des Rastarms zu liegen kommen, um den Rastarm zu blockieren und gegebenenfalls radial nach innen zu pressen.

Die Rastarme erstrecken sich vorteilhaft bis in einen proximal vom Innengewinde des Trennelements gelegenen Bereich in die axiale Richtung. Sie können sich dabei proximal an das Innengewinde anschließen. Das Innengewinde kann aber auch mindestens teilweise auf der Innenseite der Rastarme ausgebildet sein. Am Trennelement können außenseitig ein oder mehrere Mitnahmeelemente ausgebildet sein, um in Umfangsrichtung einen formschlüssigen Eingriff des Trennelements mit einem Mitnehmer zu ermöglichen. Dadurch lässt sich das Trennelement leicht vom Behälter abschrauben. Die Mitnahmeelemente können z.B. die Form von radial vorstehenden, über den Umfang verteilten, axial verlaufenden Längsrippen haben. Insbesondere können die Mitnahmeelemente eine Verzahnung entlang der Umfangsrichtung bilden.

Vorzugsweise sind die Mitnahmeelemente proximal vom Außengewinde und distal von den Rastarmen am Trennelement angeordnet. Derartige Mitnahmeelemente sind aber auch dann von Vorteil, wenn das Trennelement keine Rastarme aufweist.

Durch Kombination eines Trennelements der vorstehend genannten Art mit einem Behälter entsteht eine Austragvorrichtung. Dabei dient der Behälter zur Aufnahme eines Produkts und kann wie folgt ausgestaltet sein: mit einer umlaufenden Behälterwand, einem proximalen Behälterende, einem distalen Behälterende und einer Austragöffnung am distalen Behälterende, wobei an der Behälterwand im Bereich des distalen Behälterendes ein Außengewinde ausgebildet ist, welches um die axiale Richtung verläuft, und wobei das Außengewinde des Behälters mit dem Innengewinde des Trennelements in Eingriff bringbar ist, um das Trennelement am distalen Behälterende anzubringen. Der Behälter kann mit dem schon erwähnten Granulat, insbesondere einer Komponente eines Knochenersatzmaterials, vorbefüllt sein.

Die Austragvorrichtung kann außerdem ein Vorschubelement mit Kolben und Kolbenstange aufweisen, um das im Behälter aufgenommene Produkt nach Entfernung des Trennelements durch die Austragöffnung aus dem Behälter auszutragen. Der Kolben kann dichtend an der Innenseite der Behälterwand anliegen, um zusätzlich zu ermöglichen, Flüssigkeit durch das Trennelement hindurch in den Behälter einzusaugen.

Proximal vom Außengewinde des Behälters kann außenseitig an der Behälterwand eine Axialsicherungsstruktur ausgebildet sein, wobei der mindestens eine Rastarm des Trennelements mit der Axialsicherungsstruktur derart in Eingriff bringbar ist, dass er eine proximale Bewegung des Trennelements relativ zum Behälter behindert oder sogar vollständig blockiert (insbesondere durch einen axialen Formschluss), und wobei der Rastarm durch eine radiale Auslenkung des Rastarms nach außen außer Eingriff mit der Axialsicherungsstruktur bringbar ist. Insbesondere kann der Rastarm mit seinem schon erwähnten Hintergreifelement die Axialsicherungsstruktur axial hintergreifen, um so einen axialen Formschluss herzustellen.

Die Axialsicherungsstruktur kann z.B. einen Ringwulst umfassen, mit welchem der mindestens eine Rastarm federnd rastend in Eingriff bringbar ist. Stattdessen kann aber z.B. auch eine in die proximale Richtung abfallende Stufe oder eine Ringnut in der Behälterwand ausgebildet sein.

Alternativ oder zusätzlich zur Axialsicherungsstruktur kann proximal vom Außengewinde des Behälters außenseitig an der Behälterwand eine Drehsicherungsstruktur ausgebildet sein. Am mindestens einen Rastarm des Trennelements ist dann eine komplementäre Eingriffsstruktur ausgebildet, die derart mit der Drehsicherungsstruktur in Eingriff bringbar ist, dass sie eine Drehung des Trennelements relativ zum Behälter behindert, und die durch eine nach aussen gerichtete radiale Auslenkung des Rastarms außer Eingriff bringbar ist. Die Eingriffsstruktur kann identisch mit dem Hintergreifelement sein, d.h. das Hintergreifelement kann sowohl eine Axialsicherung als auch eine Drehsicherung des Trennelements am Behälter bewirken.

Die Drehsicherungsstruktur ist vorzugsweise proximal von der Axialsicherungsstruktur an der Behälterwand angeordnet. Sie kann aber auch gemeinsam mit der Axialsicherungsstruktur im selben Bereich der Behälterwand ausgebildet sein. Wenn z.B. die Axialsicherungsstruktur ein Ringwulst ist, kann dieser an einigen Stellen unterbrochen sein, um den axialgesicherten und drehgesicherten Eingriff einer entsprechend gestalteten Eingriffsstruktur am Rastarm zu ermöglichen.

Die Drehsicherungsstruktur kann insbesondere als Außenverzahnung ausgebildet sein, die sich mindestens über einen Teil des Umfangs der Behälterwand erstreckt. Die komplementäre Eingriffsstruktur kann als Innenverzahnung auf der Innenseite der Rastarme ausgebildet sein.

Um das Trennelement in die distale Richtung zu verschließen, kann die Austragvorrichtung mit dem schon erwähnten Verschluss versehen sein. Dieser weist dann vorzugsweise eine umlaufende Mantelwand, ein proximales Verschlussende, ein distales Verschlussende und eine Deckwand auf. Der Verschluss kann am Trennelement in einer Verschließposition angebracht werden. Die Deckwand des Verschlusses überdeckt in der Verschließposition axial das distale Ende des Trennelements. Um das Trennelement besser am Behälter zu sichern, überdeckt die Mantelwand des Verschlusses in der Verschließposition vorzugsweise den mindestens einen Rastarm radial derart, dass die Mantelwand eine radiale Auslenkung des Rastarms behindert oder sogar den Rastarm aus seiner unbelasteten Position nach innen zur Behälterwand hin auslenkt.

In der Mantelwand des Verschlusses kann ein zum proximalen Verschlussende hin offenes Innengewinde ausgebildet sein, welches um die axiale Richtung verläuft, und am Trennelement kann das schon erwähnte Außengewinde ausgebildet sein. In der Verschließposition steht dann das Außengewinde des Trennelements mit dem Innengewinde des Verschlusses in Eingriff. Das Innengewinde kann insbesondere in einem zwischen dem proximalen Verschlussende und der Deckwand gelegenen Bereich der Mantelwand angeordnet sein.

Falls das Außengewinde des Trennelements an einem Rohrstutzen ausgebildet ist, welcher innenseitig einen Hohlraum definiert, wie dies vorstehend beschrieben wurde, ist es vorteilhaft, wenn an der Deckwand des Verschlusses ein Stopfen ausgebildet ist, der sich im Inneren des Verschlusses in Richtung des proximalen Verschlussendes erstreckt. Dieser Stopfen erstreckt sich dann in der Verschließposition in den Hohlraum hinein, vorzugsweise derart, dass eine proximale Stirnfläche des Stopfens in der Verschließstellung auf dem Filterbereich aufliegt. Dadurch wird verhindert, dass Material aus dem Inneren des Behälters durch den Filterbereich hindurch in den Hohlraum gelangen kann, solange der Verschluss auf dem Trennelement angebracht ist. Das Innengewinde kann dann mindestens teilweise in einem den Stopfen umgebenden Bereich der Mantelwand ausgebildet sein.

In einigen Anwendungen kann es erwünscht sein, denselben Verschluss auch dann einzusetzen, wenn kein Trennelement vorhanden ist. Der Verschluss dient dann unmittelbar zum Verschließen des Behälters. So kann es z.B. erwünscht sein, den Verschluss erneut am Behälter anzubringen, nachdem das Granulat im Behälter mit Flüssigkeit benetzt wurde und das Trennelement entfernt wurde. Von besonderem Interesse ist dies aber in Anwendungen, bei denen von Anfang an im Behälter nicht ein zu benetzendes Granulat, sondern eine gebrauchsfertige Masse (z.B. sogenannter "Putty") aufgenommen ist, die vor dem Austragen nicht mehr mit einer Flüssigkeit gemischt zu werden braucht. Bei der Masse kann es sich insbesondere um ein Knochenersatzmaterial handeln.

In diesem Fall ist es nachteilig, wenn der Verschluss einen Stopfen aufweist, weil dieser beim Anbringen des Verschlusses in den Behälter hineinragen würde und das im distalen Endbereich des Behälters vorhandene Material verdrängen würde. Daher wird in einer Weiterbildung vorgeschlagen, den Verschluss wie folgt auszugestalten: Die Deckwand bildet auf der Rückseite des Stopfens eine distale Stirnfläche, und der Verschluss weist zwischen der distalen Stirnfläche und dem distalen Verschlussende ein zweites Innengewinde auf, welches zum distalen Verschlussende hin offen ist, um die axiale Richtung verläuft und komplementär zum Außengewinde am distalen Behälterende ausgebildet ist. Dadurch kann der Verschluss mit dem zweiten Innengewinde in einer gegenüber der Verschließposition umgekehrten Orientierung auf das Außengewinde am distalen Behälterende aufgeschraubt werden, so dass die distale Stirnfläche das distale Behälterende überdeckt. Die distale Stirnfläche kann beliebig ausgestaltet sein, insbesondere über den gesamten lichten Querschnitt des Verschlusses hinweg eben sein, so dass in dieser Orientierung kein Bestandteil des Verschlusses ins Behälterinnere ragt, wenn der Verschluss auf dem Behälter angebracht wird. Das zweite Innengewinde kann insbesondere gleich dimensioniert sein (insbesondere gleiche Gangzahl und Steigungshöhe aufweisen) wie das erste Innengewinde.

Bevorzugt erstreckt sich die Mantelwand des Verschlusses in der Verschließposition in proximaler Richtung mindestens bis zum proximalen Ende des Trennelements, so dass der Verschluss das Trennelement in der Verschließposition vollständig überdeckt. Auf diese Weise ist gewährleistet, dass das Trennelement nicht versehentlich berührt werden kann und dadurch verschmutzt wird, oder dass umgekehrt ein Benutzer nicht durch am Trennelement anhaftendes Material kontaminiert werden kann. Um das im Behälter aufgenommene Granulat mit Flüssigkeit zu benetzen, ist es zunächst erforderlich, den Verschluss zu entfernen. Dabei stellen die Rastarme am Trennelement gemeinsam mit der Mantelwand des Verschlusses sicher, dass das Trennelement am Behälter verbleibt und nicht versehentlich zusammen mit dem Verschluss entfernt wird. Die Zugabe der Flüssigkeit erfolgt anschließend durch den Filterbereich des Trennelements hindurch durch Aufsaugen oder Einspritzen.

Nachdem die Flüssigkeit in den Behälter aufgenommen wurde und mit dem im Behälter vorhandenen Granulat vermischt wurde, wird das Trennelement vom Behälter entfernt, um das fertige Produkt durch die Austragöffnung auszutragen. Um diesen Vorgang zu vereinfachen, kann die Mantelwand des Verschlusses im Bereich des distalen Verschlussendes distal von der Deckwand innenseitig einen oder mehrere Mitnehmer aufweisen. Die Mitnehmer sind dann mit den Mitnahmeelementen des Trennelements in Eingriff bringbar, indem der Verschluss in einer gegenüber der Verschließposition umgekehrten Orientierung entlang der axialen Richtung mit dem Trennelement verbunden, insbesondere auf dieses aufgeschoben, wird, so dass das Trennelement mit Hilfe des Verschlusses vom Behälter abschraubbar ist.

Eine solche Ausgestaltung ist auch dann vorteilhaft, wenn das Trennelement keine federelastischen Rastarme aufweist. Insofern wird auch ein Trennelement mit einem proximalen Ende und einem distalen Ende offenbart, aufweisend:
einen Filterbereich, der dazu ausgebildet ist, den Durchtritt eines Granulats entlang einer axialen Richtung zwischen dem proximalen Ende und dem distalen Ende zu verhindern, aber den Durchtritt von Flüssigkeit entlang der axialen Richtung zu erlauben; ein zum proximalen Ende hin offenes Innengewinde, welches um die axiale Richtung verläuft; und ein oder mehrere Mitnahmeelemente, die außenseitig auf dem Trennelement ausgebildet sind, um in Umfangsrichtung einen formschlüssigen Eingriff des Trennelements mit einem Mitnehmer eines Verschlusses zu ermöglichen.

Die Mitnahmeelemente können, wie schon erläutert, eine Verzahnung entlang der Umfangsrichtung bilden. Das Trennelement kann, wie schon erläutert, vorteilhaft ein zum distalen Ende hin offenes Außengewinde aufweisen, welches um die axiale Richtung verläuft, um einen Verschluss aufzuschrauben. Das Außengewinde kann insbesondere an einem Rohrstutzen ausgebildet sein, welcher innenseitig einen Hohlraum definiert, wobei der Hohlraum in proximaler Richtung vom Filterbereich begrenzt ist.

Es wird außerdem eine Austragvorrichtung offenbart, die ein Trennelement der vorstehend genannten Art mit den erwähnten Mitnahmeelementen aufweist, wobei die erwähnten Rastarme optional sind. Die Austragvorrichtung weist darüber hinaus eines oder mehrere der folgenden Merkmale auf:
einen Behälter zur Aufnahme eines Produkts, mit einer umlaufenden Behälterwand, einem distalen Behälterende und einer Austragöffnung am distalen Behälterende; und
einen Verschluss mit einer umlaufenden Mantelwand, einem proximalen Verschlussende, einem distalen Verschlussende und einer Deckwand.

An der Behälterwand kann im Bereich des distalen Behälterendes ein Außengewinde ausgebildet sein, welches um die axiale Richtung verläuft, wobei dieses Außengewinde des Behälters mit dem Innengewinde des Trennelements in Eingriff bringbar ist, um das Trennelement am distalen Behälterende anzubringen. Der Verschluss kann derart am Trennelement anbringbar sein, dass die Deckwand des Verschlusses in einer Verschließposition das distale Ende des Trennelements axial überdeckt. Die Mantelwand des Verschlusses kann distal von der Deckwand innenseitig einen oder mehrere Mitnehmer aufweisen. Die Mitnehmer können mit den Mitnahmeelementen des Trennelements in Eingriff bringbar sein, indem der Verschluss in einer gegenüber der Verschließposition umgekehrten Orientierung entlang der axialen Richtung mit dem Trennelement verbunden, insbesondere auf dieses aufgeschoben, wird, so dass das Trennelement mit Hilfe des Verschlusses vom Behälter abschraubbar ist.

Die Mitnehmer und Mitnahmeelemente können derart miteinander in Eingriff bringbar sein, dass sie bezüglich der Umfangsrichtung eine formschlüssige Verbindung miteinander eingehen. Die Mitnehmer können dazu z.B. Längsrippen oder Nocken umfassen, und die Mitnahmeelemente können dazu komplementäre Längsnuten umfassen, oder umgekehrt. Zusätzlich können Mitnehmer und Mitnahmeelemente auch eine axial gesicherte Verbindung eingehen, z.B. durch Reibschluss, durch eine Schnappverbindung, durch eine Bajonettverbindung usw. Im Fall von Mitnahmeelementen in Form von Längsnuten kann sich z.B. an das proximale Ende dieser Längsnuten jeweils eine kurze Quernut anschließen, um eine Bajonettverbindung mit nockenförmigen Mitnehmern zu ermöglichen. So kann sichergestellt werden, dass der Verschluss und das Trennelement beim Entfernen vom Behälter eine Einheit bilden und das Trennelement nach dem Entfernen nicht aus dem Verschluss herausfällt. Gleichzeitig wird verhindert, dass der Benutzer jemals das Trennelement berühren muss. Eine Kontamination zwischen Benutzer und Trennelement wird so in beide Richtungen vermieden.

Es wird auch ein Verfahren zur Herstellung und zum Austragen eines Produkts mit einer Austragvorrichtung der vorstehend genannten Art offenbart, wobei das Verfahren aufweist:
Bereitstellen der Austragvorrichtung in einem Zustand, in dem der Behälter mit einem Granulat befüllt ist, das Trennelement über der Austragöffnung des Behälters angebracht ist, und der Verschluss in der Verschließposition am Trennelement angebracht ist,
Entfernen des Verschlusses vom Trennelement, während das Trennelement am Behälter verbleibt;
Aufnehmen einer Flüssigkeit durch das Trennelement hindurch in den Behälter, um das Granulat mit der Flüssigkeit zu benetzen und so ein austragfertiges Produkt herzustellen;
Verbinden des Verschlusses mit dem Trennelement in einer gegenüber der Verschließposition umgekehrten Orientierung, insbesondere durch Aufschieben des Verschlusses auf das Trennelement;

Abschrauben des Trennelements vom Behälter mit Hilfe des Verschlusses; und Austragen des austragfertigen Produkts durch die Austragöffnung des Behälters. Wie erläutert, kann es auch erwünscht sein, den Behälter schon von Anfang an mit einem austragfertigen Produkt, insbesondere einem Knochenersatzmaterial, vorzubefüllen. Das Trennelement kann dann ganz entfallen. Um sicherzustellen, dass der Verschluss wahlweise auch direkt auf den Behälter aufgeschraubt werden kann, sind das Außengewinde des Trennelements und das Außengewinde des Behälters vorzugsweise gleich dimensioniert, so dass der Verschluss mit seinem Innengewinde wahlweise auf das Trennelement oder direkt auf den Behälter aufschraubbar ist. In anderen Worten kann das Innengewinde des Verschlusses wahlweise mit dem Außengewinde des Trennelements oder dem Außengewinde des Behälters in Eingriff bringbar sein. In diesem Fall wird meist zwischen der Behälterwand und der Mantelwand des Verschlusses ein radialer Zwischenraum entstehen, wenn der Verschluss direkt auf den Behälter aufgeschraubt ist. Dieser Zwischenraum entsteht insbesondere in einem proximal vom Gewinde gelegenen Bereich. Um zu verhindern, dass Schmutz in diesen Zwischenraum gelangt, kann an der Behälterwand außenseitig proximal vom Außengewinde eine Überbrückungsstruktur, vorzugsweise in Form eines Ringwulstes, ausgebildet sein, welche radial so weit vorsteht, dass sie den radialen Zwischenraum überbrückt. Diese Überbrückungsstruktur ist insbesondere vorzugsweise proximal von der Axialsicherungsstruktur und/oder Drehsicherungsstruktur angeordnet. Derselbe Behälter und derselbe Verschluss können also ohne jegliche konstruktive Änderung wahlweise mit oder ohne Trennelement verwendet werden. Dies trägt zu wesentlichen Kosteneinsparungen bei, da bei der Herstellung des Behälters und des Verschlusses dieselben Spritzgussformen unabhängig davon verwendet werden können, ob ein Trennelement vorgesehen wird. Behälter und Verschluss sind in diesem Fall quasi selbst dann für eine Verwendung mit Trennelement ausgestaltet, wenn dieses nicht benötigt wird.

Dementsprechend wird also auch eine Austragvorrichtung offenbart, aufweisend einen Behälter zur Aufnahme eines Produkts, mit einer umlaufenden Behälterwand, einem proximalen Behälterende, einem distalen Behälterende und einer Austragöffnung am distalen Behälterende; und einen Verschluss mit einer umlaufenden Mantelwand, einem proximalen Verschlussende, einem distalen Verschlussende und einer Deckwand, wobei an der Behälterwand im Bereich des distalen Behälterendes ein Außengewinde ausgebildet ist, welches um eine axiale Richtung verläuft, wobei in der Mantelwand in einem zwischen dem proximalen Verschlussende und der Deckwand gelegenen Bereich ein Innengewinde ausgebildet ist, welches um die axiale Richtung verläuft, wobei das Außengewinde des Behälters mit dem Innengewinde des Verschlusses in Eingriff steht, wobei die Deckwand des Verschlusses die Austragöffnung des Behälters überdeckt, wobei ein radialer Zwischenraum zwischen der Behälterwand und der Mantelwand des Verschlusses besteht, und wobei an der Behälterwand außenseitig proximal vom Außengewinde eine Überbrückungsstruktur, vorzugsweise in Form eines Ringwulstes, ausgebildet ist, welche radial so weit vorsteht, dass sie den radialen Zwischenraum überbrückt, um den Eintritt von Schmutz in den radialen Zwischenraum zu verhindern.

Insbesondere können an der Mantelwand außenseitig die schon erwähnte Axialsicherungsstruktur und/oder Drehsicherungsstruktur ausgebildet sein, und die Überbrückungsstruktur ist dann vorzugsweise proximal von der Axialsicherungsstruktur und/oder Drehsicherungsstruktur an der Behälterwand angeordnet, wie dies oben schon beschrieben wurde.

Vorzugsweise grenzt die Überbrückungsstruktur unmittelbar an das proximale Verschlussende an oder ist vollständig von der Mantelwand des Verschlusses überdeckt, und die Mantelwand des Verschlusses kann an der Überbrückungsstruktur anliegen. Dies kann in dichtender oder nicht dichtender Weise geschehen.

In üblicher Weise kann der Behälter außenseitig am proximalen Behälterende eine erste Fingerauflage aufweisen. Diese dient als proximale Auflage für Zeigefinger und Mittelfinger, wenn der Benutzer den Behälter zwischen Zeigefinger und Mittelfinger ergreift und mit dem Daumen das Vorschubelement in distaler Richtung aus dem Behälter vorschiebt. Das Vorschubelement kann einen Daumenring aufweisen, um nicht nur einen solchen distalen Vorschub zu ermöglichen, sondern auch zu ermöglichen, das Vorschubelement mit dem Daumen zurückzuziehen. Um dabei ein Abrutschen von Zeigefinger und Mittelfinger zu verhindern, kann der Behälter distal von der ersten Fingerauflage außenseitig eine zweite Fingerauflage aufweisen, um ein Abrutschen von Fingern eines Benutzers entlang der distalen Richtung zu behindern.

Es wird außerdem ein Verschluss offenbart, welcher eine axiale Richtung, ein proximales Verschlussende und ein distales Verschlussende definiert. Der Verschluss weist auf:
eine um die axiale Richtung umlaufende Mantelwand;
eine Deckwand, die eine distale Stirnfläche bildet;
einen Stopfen, der sich im Inneren des Verschlusses ausgehend von der Deckwand in die proximale Richtung erstreckt, wobei der Stopfen an seinem freien proximalen Ende eine proximale Stirnfläche bildet,
ein erstes Innengewinde, das um die axiale Richtung verläuft und zum proximalen Verschlussende hin offen ist, und
ein zweites Innengewinde, das in einem zwischen der distalen Stirnfläche und dem distalen Verschlussende gelegenen Bereich ausgebildet ist, um die axiale Richtung verläuft und zum distalen Verschlussende hin offen ist.

Wie oben vorstehend erläutert wurde, kann ein solcher Verschluss wahlweise dazu verwendet werden, in einer ersten Orientierung mit Hilfe des ersten Innengewindes auf einem Trennelement angebracht zu werden, das distal vom Filterbereich einen Rohrstutzen mit Außengewinde aufweist, oder er kann dazu verwendet werden, in einer zweiten Orientierung direkt auf einem vorbefüllten Behälter angebracht zu werden, ohne dass der Stopfen das im Behälter vorhandene Material verdrängt. Das erste Innengewinde ist vorzugsweise mindestens teilweise in einem den Stopfen umgebenden Bereich der Mantelwand ausgebildet, damit es in ein Außengewinde am Rohrstutzen eingreifen kann.

Wie vorstehend genauer erläutert, kann die die Mantelwand distal von der distalen Stirnfläche, insbesondere im Bereich des distalen Verschlussendes, innenseitig einen oder mehrere Mitnehmer aufweisen, um das Abschrauben des Trennelements zu ermöglichen.

Es wird außerdem vorgeschlagen, die Austragvorrichtung in einer speziellen Verpackung unterzubringen, die noch eine oder mehrere weitere Zusatzfunktionen bereitstellt. Insbesondere wird vorgeschlagen, eine Kombination einer Austragvorrichtung der vorstehend genannten Art mit einer die Austragvorrichtung aufnehmende Verpackung bereitzustellen. Die Verpackung weist einen Träger auf, der eine horizontale Oberseite definiert. Beim Träger kann es sich insbesondere um ein thermogeformtes Folienformteil handeln, wie es im Stand der Technik häufig als Bestandteil von sogenannten Blisterverpackungen eingesetzt wird. Um die Verpackung zu verschließen, kann die Verpackung außerdem eine Verschlussschicht oder eine andere Art von Gegenstück zum Träger aufweisen, die auf die horizontale Oberseite aufgesiegelt ist, z.B. eine Verschlussschicht aus Kunststoff, Papier oder einem Verbundmaterial. In dem Träger ist eine nach oben offene Aufnahmevertiefung zur liegenden Aufnahme der Austragvorrichtung ausgebildet. Um die Handhabung der Austragvorrichtung zu vereinfachen und besonders intuitiv zu gestalten, ist vorzugsweise im Träger eine Verschlusshaltevertiefung ausgebildet, die komplementär zum Verschluss dimensioniert ist. Der Verschluss ist dann derart orientiert in die Verschlusshaltevertiefung einsteckbar, dass das distale Verschlussende nach oben weist. Der Verschluss kann in dieser Orientierung insbesondere reibschlüssig in der Verschlusshaltevertiefung aufnehmbar sein. Dabei kann die Verschlusshaltevertiefung derart ausgebildet und dimensioniert sein, dass sie eine seitliche Klemmkraft auf den Verschluss ausübt, wenn dieser in der entsprechenden Orientierung in die Verschlusshaltevertiefung eingesetzt ist. Der Verschluss kann insbesondere kippsicher in der Verschlusshaltevertiefung aufnehmbar sein. Die Verschlusshaltevertiefung kann separat von der Aufnahmevertiefung ausgebildet sein, oder sie kann als Bereich der Aufnahmevertiefung ausgebildet sein und z.B. durch eine bereichsweise Aufweitung der Aufnahmevertiefung gebildet sein. Um eine besonders intuitive Handhabung zu ermöglichen, kann benachbart zur Verschlusshaltevertiefung eine Markierung auf dem Träger angebracht sein, die durch grafische Elemente oder Schriftzeichen darauf hinweist, dass die entsprechende Vertiefung zur Aufnahme des Verschlusses vorgesehen ist.

Im Träger kann außerdem eine nach oben offene, separate Fluidreservoirvertiefung ausgebildet ist, die es ermöglicht, eine Flüssigkeit wie z.B. Kochsalzlösung oder Blut aufzunehmen. Auf diese Weise kann die Flüssigkeit mit der Austragvorrichtung bequem aufgesaugt werden, nachdem der Verschluss von der Austragvorrichtung entfernt wurde, um ein Granulat in der Austragvorrichtung mit der Flüssigkeit zu benetzen. Es wird keine separate Schale für die Flüssigkeit benötigt. Die Fluidreservoirvertiefung ist vorzugsweise separat von der Aufnahmevertiefung und der Verschlusshaltevertiefung ausgebildet. Auch hier kann die Handhabung besonders intuitiv gestaltet werden, indem benachbart zur Fluidreservoirvertiefung eine Markierung auf den Träger angebracht ist, die durch grafische Elemente oder Schriftzeichen darauf hinweist, dass die entsprechende Vertiefung zur Aufnahme einer Flüssigkeit vorgesehen ist. Die Austragvorrichtung kann in an sich bekannter Weise ein Vorschubelement aufweisen, um das im Behälter aufgenommene Produkt nach Entfernung des Trennelements durch die Austragöffnung durch Vorschieben des Vorschubelements aus dem Behälter auszutragen. Um zu ermöglichen, mit dem Vorschubelement auch auf einfache Weise Flüssigkeit in den Behälter einzusaugen, kann das Vorschubelement einen Daumenring aufweisen.

Je nach Art und Menge der Befüllung kann es erwünscht sein, das Vorschubelement relativ zum Behälter in unterschiedlichen Vorschubstellungen in der Verpackung zu fixieren. So kann es z.B. erwünscht sein, das Vorschubelement in einer Zwischenstellung ungefähr auf halbem Weg zwischen einer Ausgangsstellung, in der das Vorschubelement maximal in die proximale Richtung herausgezogen ist, und einer Endstellung, in der das Vorschubelement maximal in die distale Richtung eingeschoben wurde, zu fixieren. Dies ist z.B. dann der Fall, wenn der Behälter mit einem Granulat vorbefüllt ist, das vor der Verabreichung noch mit einer Flüssigkeit hydriert werden muss. Andererseits kann es erwünscht sein, das Vorschubelement stattdessen auch in der Ausgangsstellung zu fixieren. Dies ist zum Beispiel dann der Fall, wenn der Behälter mit einem gebrauchsfertigen Produkt befüllt ist. Um für beide Anforderungen gewappnet zu sein, kann die Aufnahmevertiefung einen ersten Vertiefungsbereich für die Aufnahme des Daumenrings aufweisen, wobei der Daumenring im ersten Vertiefungsbereich zu liegen kommt, wenn sich das Vorschubelement relativ zum Behälter in einer ersten Vorschubstellung befindet, und die Aufnahmevertiefung kann einen zweiten Vertiefungsbereich für die Aufnahme des Daumenrings aufweisen, wobei der Daumenring im zweiten Vertiefungsbereich zu liegen kommt, wenn sich das Vorschubelement in einer zweiten Vorschubstellung befindet, die sich von der ersten Vorschubstellung unterscheidet. Ein Verfahren zur Herstellung und zum Austragen eines Produkts mit einer Austragvorrichtung und Verpackung der vorstehend genannten Art weist auf: Bereitstellen der Austragvorrichtung in einem Zustand, in dem der Behälter mit einem Granulat befüllt ist, das Trennelement über der Austragöffnung des Behälters angebracht ist, und der Verschluss in der Verschließposition am Trennelement angebracht ist,
Entfernen des Verschlusses vom Trennelement, während das Trennelement am Behälter verbleibt;
Einsetzen des Verschlusses in die Verschlusshaltevertiefung derart, dass das distale Verschlussende nach oben weist;
Bereitstellen einer Flüssigkeit in der Fluidreservoirvertiefung;
Aufnehmen der Flüssigkeit durch das Trennelement hindurch in den Behälter, um das Granulat mit der Flüssigkeit zu benetzen und so ein austragfertiges Produkt herzustellen;
Aufnehmen des Verschlusses aus der Verschlusshaltevertiefung mittels der Austragvorrichtung, so dass der Verschluss in einer gegenüber der Verschließposition umgekehrten Orientierung mit dem Trennelement verbunden wird;

Abschrauben des Trennelements vom Behälter mit Hilfe des Verschlusses; und Austragen des austragfertigen Produkts durch die Austragöffnung des Behälters. Auf diese Weise wird nicht nur verhindert, dass der Benutzer jemals das Trennelement berühren muss, sondern es wird auch die Wahrscheinlichkeit einer versehentlichen Berührung stark vermindert, weil der Benutzer den Verschluss nicht in die Hand zu nehmen braucht, um ihn nach der Aufnahme der Flüssigkeit mit dem Trennelement zu verbinden.

In einigen Anwendungen kann es erwünscht sein, die Flüssigkeit nicht aus einem offenen Reservoir wie einer Schale oder einer Vertiefung in einem Träger aufzunehmen, sondern die Flüssigkeit in einem geschlossenen Reservoir wie einer Spritze bereitzustellen. Es wird daher ein Trennelement vorgeschlagen, das die Aufnahme der Flüssigkeit aus einem geschlossenen Reservoir wie einer Spritze erleichtert.

Das Trennelement bildet ein proximales Ende und ein distales Ende und definiert eine axiale Richtung, die vom proximalen zum distalen Ende verläuft. Es weist auf: einen Filterbereich, der dazu ausgebildet ist, den Durchtritt eines Granulats entlang der axialen Richtung zwischen dem proximalen Ende und dem distalen Ende zu verhindern, aber den Durchtritt von Flüssigkeit entlang der axialen Richtung zu erlauben; und ein zum proximalen Ende hin offenes Innengewinde, welches um die axiale Richtung verläuft; und einen distal vom Filterbereich gelegenen Anschlussbereich, der einen zum distalen Ende hin offenen weiblichen Luerkonus bildet.

Viele geschlossene Reservoirs, insbesondere handelsübliche Spritzen, weisen einen Auslassbereich auf, der einen männlichen Luerkonus bildet. Mit der vorgeschlagenen Ausgestaltung des Trennelements wird eine Verbindung zwischen einem Reservoir mit männlichem Luerkonus und dem Trennelement erleichtert, indem sich der männliche Luerkonus des Reservoirs einfach in den weiblichen Luerkonus des Trennelements einstecken lässt.

Als weiblicher Luerkonus wird ein Innenkegel mit einer Neigung von 6% zur axialen Richtung bezeichnet. Luerverbindungen sind in der Norm ISO 594-1 :2003-08-30 ("Conical fittings with a 6% (Luer) taper for syringes, needles and certain other medical equipment; Part 1 : General requirements") genormt. Diese Norm wird durch Verweis vollständig in die vorliegende Offenbarung aufgenommen. Wenn im vorliegenden Dokument auf eine Luerverbindung oder einen Luerkonus Bezug genommen wird, ist immer eine entsprechende Struktur gemäß der genannten Norm gemeint.

Das Trennelement kann in seinem Anschlussbereich außerdem außenseitig mindestens ein Eingriffselement aufweisen, das für einen Eingriff mit einem Innengewinde eines Aufsatzes ausgebildet ist. Beim Eingriffselement kann es sich insbesondere um einen Bajonettflansch oder ein kurzes Gewindesegment handeln. Das Eingriffselement kann insbesondere gemäß der Norm ISO 594-2:2003-08-30 ("Conical fittings with 6% (Luer) taper for syringes, needles and certain other medical equipment; Part 2: Lock fittings") ausgestaltet sein. Auch diese Norm wird durch Verweis vollständig in die vorliegende Offenbarung aufgenommen.

Es wird außerdem eine Austragvorrichtung vorgeschlagen, die ein Trennelement der vorstehend genannten Art aufweist. Die Austragvorrichtung weist außerdem einen Behälter zur Aufnahme eines Produkts auf, mit einer umlaufenden Behälterwand, einem proximalen Behälterende, einem distalen Behälterende und einer Austragöffnung am distalen Behälterende. An der Behälterwand ist im Bereich des distalen Behälterendes ein Außengewinde ausgebildet, welches um die axiale Richtung verläuft. Das Außengewinde des Behälters kann mit dem Innengewinde des Trennelements in Eingriff gebracht werden, um das Trennelement am distalen Behälterende anzubringen.

Die Austragvorrichtung kann außerdem um einen Verschluss ergänzt werden. Der Verschluss weist eine Deckwand, eine Mantelwand, einen sich von der Deckwand in eine proximale Richtung erstreckenden Stopfen, der einen männlichen Luerkonus bildet, sowie ein an der Mantelwand ausgebildetes, zum proximalen Ende hin offenes Innengewinde auf, wobei das Innengewinde insbesondere proximal von der Deckwand angeordnet ist. Das Innengewinde ist mit dem vorstehend erwähnten, außenseitig am Anschlussbereich des Trennelements ausgebildeten Eingriffselement in Eingriff bringbar.

Ein Verfahren zur Herstellung und zum Austragen eines Produkts mit einer Austragvorrichtung der vorstehend genannten Art weist auf:
Bereitstellen der Austragvorrichtung in einem Zustand, in dem der Behälter mit einem Granulat befüllt ist, das Trennelement über der Austragöffnung des Behälters angebracht ist, und der Verschluss in einer Verschließposition am Trennelement angebracht ist,
Entfernen des Verschlusses vom Trennelement, während das Trennelement am Behälter verbleibt;
Verbinden eines Fluidreservoirs mit dem Trennelement, wobei das Fluidreservoir eine Flüssigkeit enthält und einen Auslassbereich aufweist, der einen männlichen Luerkonus bildet, und wobei der Auslassbereich in den Anschlussbereich des Trennelements eingeführt wird, um das Fluidreservoir mit dem Trennelement zu verbinden;
Aufnehmen von Flüssigkeit aus dem Fluidreservoir durch das Trennelement hindurch in den Behälter, um das Granulat mit der Flüssigkeit zu benetzen und so ein austragfertiges Produkt herzustellen;
Entfernen des Fluidreservoirs und des Trennelements vom Behälter; und Austragen des austragfertigen Produkts durch die Austragöffnung des Behälters.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
Fig. 1 eine Explosionsdarstellung einer Austragvorrichtung gemäß einer ersten Ausführungsform, mit Behälter, Trennelement und Verschluss;
Fig. 2 eine Seitenansicht der Austragvorrichtung der Figur 1;
Fig. 3 einen zentralen Längsschnitt durch die Austragvorrichtung der Figur 1;
Fig. 4 eine Detailansicht des Bereichs A der Figur 3;
Fig. 5 eine Detailansicht des distalen Endes des Behälters der Austragvorrichtung der Figur 1;
Fig. 6 eine perspektivische Ansicht des Trennelements der Austragvorrichtung der Figur 1;
Fig. 7 einen zentralen Längsschnitt durch das Trennelement der Figur 6;
Fig. 8 eine perspektivische Ansicht des Verschlusses der Austragvorrichtung gemäß Figur 1;
Fig. 9 einen zentralen Längsschnitt durch den Verschluss der Figur 8;
Fig. 10 einen zentralen Längsschnitt durch den distalen Endbereich der Austragvorrichtung der Figur 1, wobei der Verschluss in umgekehrter Orientierung aufgesetzt ist;
Fig. 11 eine Schnittansicht in der Ebene B- B der Figur 10;
Fig. 12 einen zentralen Längsschnitt durch den distalen Endbereich der Austragvorrichtung der Figur 1, wobei das Trennelement weggelassen wurde und der Verschluss unmittelbar auf den Behälter aufgeschraubt wurde;
Fig. 13 eine perspektivische Ansicht des distalen Endes des Behälters einer Austragvorrichtung gemäß einer zweiten Ausführungsform;
Fig. 14 eine perspektivische Ansicht des Trennelements der Austragvorrichtung der zweiten Ausführungsform;
Fig. 15 eine Seitenansicht der Austragvorrichtung gemäß der zweiten Ausführungsform;
Fig. 16: eine Schnittansicht in der Ebene C- C der Figur 15;
Fig. 17: eine perspektivische Ansicht einer Austragvorrichtung gemäß einer dritten Ausführungsform
Fig. 18: einen zentralen Längsschnitt durch die Austragvorrichtung der dritten Ausführungsform;
Fig. 19: eine perspektivische Ansicht des Trennelements der Austragvorrichtung der dritten Ausführungsform;
Fig. 20: einen zentralen Längsschnitt durch das Trennelements der Figur 19;
Fig. 21: einen zentralen Längsschnitt durch den Verschluss der Austragvorrichtung der dritten Ausführungsform;
Fig. 22: einen zentralen Längsschnitt durch den distalen Endbereich der Austragvorrichtung der dritten Ausführungsform, wobei sich der Verschluss in einer Verschließstellung befindet;
Fig. 23: einen zentralen Längsschnitt durch den distalen Endbereich der Austragvorrichtung der dritten Ausführungsform, wobei der Verschluss abgeschabt wurde und in umgekehrter Orientierung wieder aufgesetzt wurde;
Fig. 24: eine Schnittansicht in der Ebene D- D der Figur 23;
Fig. 25: einen zentralen Längsschnitt durch den Verschluss einer Austragvorrichtung gemäß einer vierten Ausführungsform;
Fig. 26: einen zentralen Längsschnitt durch den distalen Endbereich der Austragvorrichtung der vierten Ausführungsform, wobei sich der Verschluss in einer Verschließstellung befindet;
Fig. 27: einen zentralen Längsschnitt durch den distalen Endbereich der Austragvorrichtung der vierten Ausführungsform, wobei das Trennelement entfernt wurde und der Verschluss in umgekehrter Orientierung auf das freie Ende des Behälters aufgeschraubt wurde;
Fig. 28: einen zentralen Längsschnitt durch den distalen Endbereich der Austragvorrichtung der vierten Ausführungsform, wobei der Verschluss in umgekehrter Orientierung auf das Trennelement aufgeschoben wurde;
Fig. 29: einen zentralen Längsschnitt durch den distalen Endbereich einer Austragvorrichtung gemäß einer fünften Ausführungsform;
Fig. 30: eine perspektivische Ansicht eines Verpackungselements mit darin aufgenommener Austragvorrichtung;
Fig. 31: eine Draufsicht auf das Verpackungselement mit der darin aufgenommenen
   Austragvorrichtung gemäß Figur 30;
Fig. 32: eine Draufsicht auf das Verpackungselement mit der darin aufgenommenen
Austragvorrichtung in einer zweiten Stellung, mit weiter herausgezogenem Vorschubelement;
Fig. 33: eine perspektivische Ansicht des Verpackungselements mit eingesteckten Verschluss;
Fig. 34: eine perspektivische Ansicht des Verpackungselements und der Austragvorrichtung zur Illustration der Verwendung zur Aufnahme einer Flüssigkeit in die Austragvorrichtung;
Fig. 35: eine perspektivische Ansicht des Verpackungselements und der Austragvorrichtung zur Illustration der Verbindung der Austragvorrichtung mit dem Verschluss, nachdem eine Flüssigkeit in die Austragvorrichtung aufgenommen wurde;
Fig. 36: eine perspektivische Ansicht einer Austragvorrichtung gemäss einer sechsten Ausführungsform;
Fig. 37: einen zentralen Längsschnitt durch die Austragvorrichtung der Figur 36; und
Fig. 38: einen zentralen Längsschnitt durch die Austragvorrichtung der Figur 36 in einem Zustand, in dem diese mit einer handelsüblichen Spritze verbunden wurde.

### BESCHREIBUNG BEVORZUGTER AUSFÜHRUNGSFORMEN

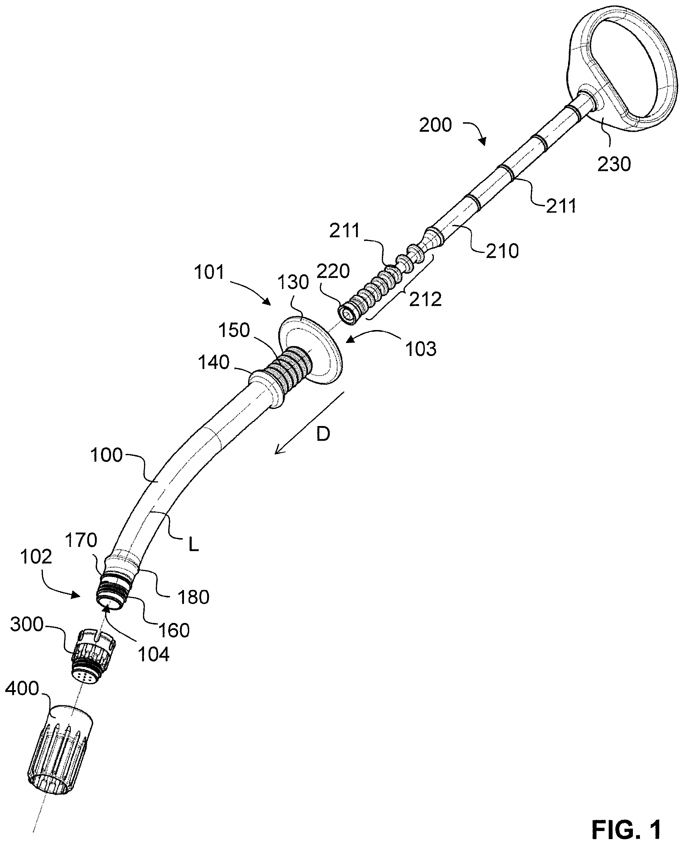

In den Figuren 1 bis 12 ist ein erstes Ausführungsbeispiel einer Austragvorrichtung in verschiedenen Ansichten illustriert. Die Austragvorrichtung umfasst einen Behälter 100, ein Vorschubelement 200, um ein im Behälter aufgenommenes Produkt entlang einer distalen Richtung D auszutragen, ein Trennelement 300 sowie einen Verschluss 400.

Die distale Richtung D ist als diejenige Richtung definiert, entlang welcher sich das Vorschubelement 200 in den Behälter 100 hinein bewegt, um das Produkt aus dem Behälter 100 auszutragen. Die dazu entgegengesetzte Richtung wird als proximale Richtung bezeichnet.

Der in den Figuren 1-5 besonders gut erkennbare Behälter 100 weist eine umlaufende Behälterwand 110, welche einen Behälterinnenraum 120 begrenzt, ein offenes proximales Behälterende 101 und ein offenes distales Behälterende 102 auf. Der Behälterinnenraum ist zumindest annähernd von kreiszylindrischer Form, wobei die Zylinderachse eine zentrale Längsachse L definiert. Der lichte Querschnitt des Behälters 100 ist dadurch entlang der gesamten Strecke vom proximalen Behälterende 101 bis zum distalen Behälterende 102 mindestens annähernd konstant, wobei gegebenenfalls das proximale Behälterende etwas aufgeweitet sein kann, um das Einschieben des Vorschubelements 200 zu erleichtern, und wobei in diesem Bereich eine Rückzugsicherung ausgebildet sein kann, um das vollständige Herausziehen des Vorschubelements 200 aus dem Behälter 100 zu verhindern. Das Vorschubelement 200 lässt sich am proximalen Behälterende 101 durch eine Einführöffnung 103 in den Behälter 100 einschieben. Die zentrale Längsachse L des Behälters 100 verläuft vom proximalen Ende 101 zum distalen Ende 102 hin zunächst gerade, krümmt sich dann aber in einem Krümmungsbereich 105 zunehmend, so dass der proximale Endbereich und der distale Endbereich des Behälters relativ zueinander in einem Krümmungswinkel α von etwa 30° verlaufen.

Das distale Behälterende 102 bildet eine Austragöffnung 104, durch welche das Produkt aus dem Behälter 100 ausgetragen werden kann. Im Bereich des distalen Behälterendes 102 ist auf der Außenseite der Behälterwand 110 ein Außengewinde 160 ausgebildet, welches eine Verbindung des Behälters 100 mit dem Trennelement 300 oder mit dem Verschluss 400 ermöglicht. Proximal vom Außengewinde ist auf der Außenseite der Behälterwand 110 integral eine Axialsicherungsstruktur 170 in Form eines Ringwulstes ausgebildet, deren Funktion nachstehend noch näher erläutert wird. Proximal beabstandet von der Axialsicherungsstruktur 170 ist auf der Außenseite der Behälterwand 1 10 integral eine Überbrückungsstruktur 180 in Form eines weiteren, radial weiter nach außen vorstehenden Ringwulstes ausgebildet, deren Funktion nachstehend ebenfalls noch näher erläutert wird.

Am proximalen Behälterende 101 ist eine erste Fingerauflage 130 in Form eines umlaufenden Flansches ausgebildet. Stattdessen können beispielsweise auch zwei Halteflügel vorgesehen sein. Um ein Abrutschen der Finger des Benutzers zu verhindern, wenn der Benutzer den Behälter zwischen Zeigefinger und Mittelfinger ergreift und mit dem Daumen das Vorschubelement 200 in proximaler Richtung aus dem Behälter herausziehen möchte, ist an der Behälterwand 110 außenseitig eine zweite Fingerauflage 140 ausgebildet. Die zweite Fingerauflage 140 ist distal von der ersten Fingerauflage in einem Abstand von dieser angeordnet, wobei dieser Abstand in etwa der Dicke eines Fingers entspricht, typischerweise ca. 1.5-3 cm. Im vorliegenden Beispiel ist die zweite Fingerauflage 140 als ein Ringwulst ausgebildet, der um ca. 1-2 mm radial aus der Behälterwand nach außen vorsteht. Zwischen der ersten und der zweiten Fingerauflage ist in der Behälterwand eine Rillenstruktur 150 ausgebildet, die zusätzlich einem Abrutschen der Finger entgegenwirkt. Die Fingerauflagen 130, 140 und die Rillenstruktur 150 sind integral mit der Behälterwand 110 ausgebildet. Das Vorschubelement 200 ist in den Figuren 1-3 erkennbar. Es weist eine Kolbenstange 210 auf, an deren distalem Ende ein Kolben 220 zum Vorschieben eines im Behälter 100 aufgenommenen Produkts ausgebildet ist. Der Kolben 220 liegt dabei umlaufend dichtend an der Innenseite der Behälterwand 110 an. Um die Reibung zwischen der Behälterwand 1 10 und der Kolbenstange 210 zu reduzieren, weist die Kolbenstange 210 einen Außendurchmesser auf, welcher geringer als der Innendurchmesser des Behälters 100 ist. Um dennoch eine gute Führung der Kolbenstange 210 im Behälterinnenraum 120 zu gewährleisten, weist die Kolbenstange 210 radial nach außen vorstehende, umlaufende, ringförmige Führungswülste 211 auf, deren Außendurchmesser dem Innendurchmesser des Behälters 100 entspricht, so dass die Führungswülste 211 innenseitig an der Behälterwand 110 anliegen. Damit die Kolbenstange 210 auch der Krümmung des Behälters 100 gut folgen kann, ist der Außendurchmesser der Kolbenstange 210 in einem distalen Bereich 212 zusätzlich reduziert, und die Führungswülste 211 sind in diesem Bereich dichter angeordnet. Dies verleiht der Kolbenstange 210 im distalen Bereich 212 zusätzliche Flexibilität, ohne die laterale Führung der Kolbenstange 210 zu beeinträchtigen.

Am proximalen Ende der Kolbenstange 210 ist ein Daumenring 230 ausgebildet, der genügend groß ist, damit der Daumen eines Benutzers eingeführt werden kann. Durch die Ausbildung der Daumenauflage als Ring wird es möglich, das Vorschubelement 200 mit Hilfe des Daumens nicht nur in die distale Richtung D in den Behälter 100 einzuschieben, sondern auch in die proximale Richtung aus dem Behälter 100 herauszuziehen, während der Behälter zwischen Zeigefinger und Mittelfinger derselben Hand gehalten wird. Auf diese Weise kam insbesondere eine Flüssigkeit in den Behälter eingesaugt werden.

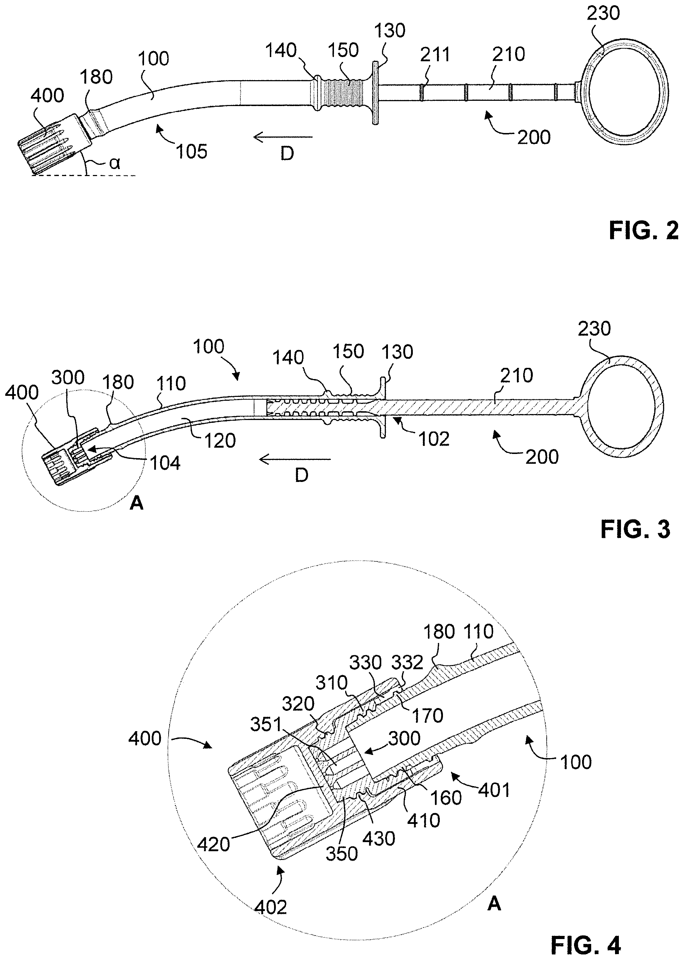

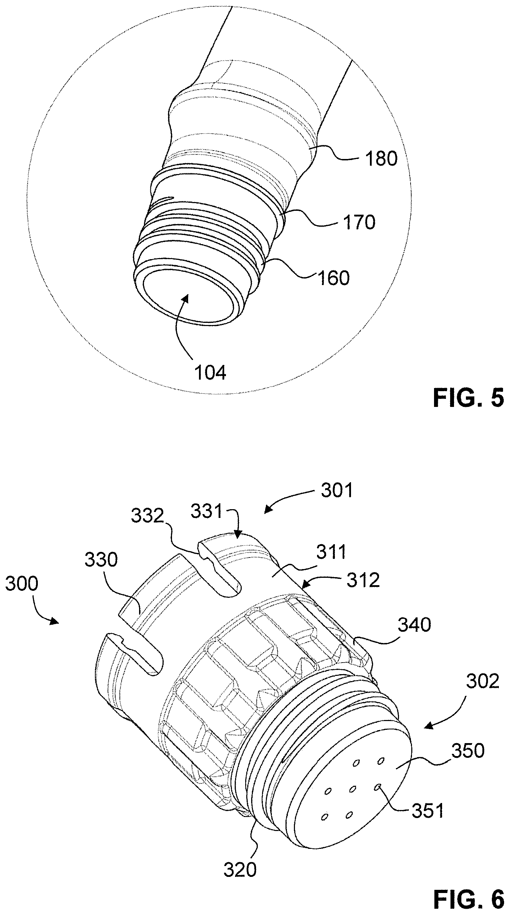

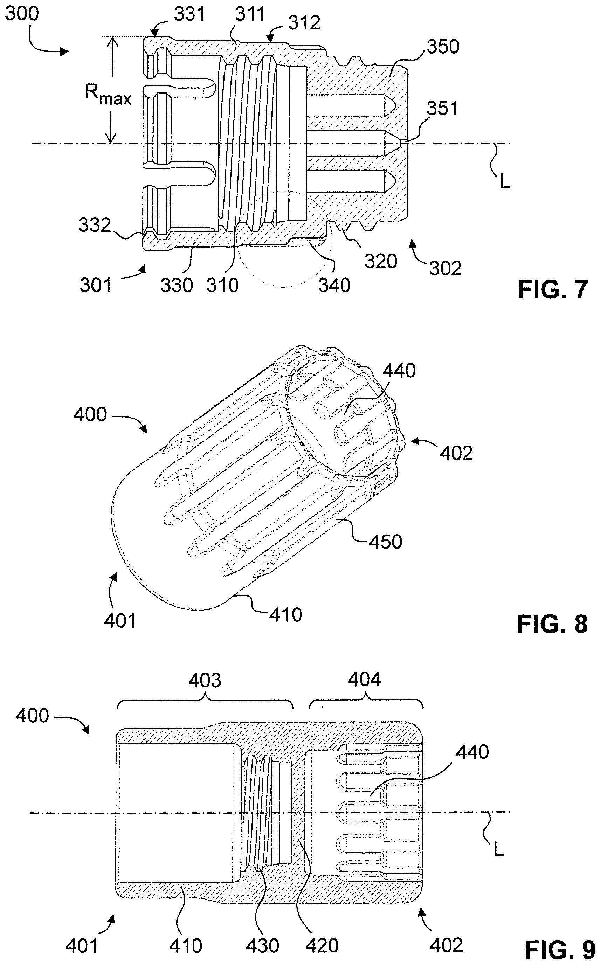

Das Trennelement 300 ist in den Figuren 6 und 7 besonders gut erkennbar. Es weist einen Filterbereich 350 auf, der dazu ausgebildet ist, den Durchtritt eines Granulats entlang der distalen Richtung D zu verhindern, aber den Durchtritt von Flüssigkeit entlang der proximalen Richtung zu erlauben. Der Filterbereich 350 weist dazu eine Vielzahl von axialen Durchlassöffnungen 351 auf, die parallel zur Längsachse L verlaufen. Die distale Stirnfläche des Filterbereichs 350 bildet das distale Ende 302 des Trennelements 300. Auf der umlaufenden Mantelfläche des Filterbereichs 350 ist ein Außengewinde 320 ausgebildet, das sich um die zentrale Längsachse herum erstreckt und das zum distalen Ende 302 hin offen ist, damit von diesem Ende her der Verschluss 400 auf das Trennelement aufgeschraubt werden kann. In proximaler Richtung schließt sich an den Filterbereich 350 ein umlaufender Mantelwandbereich 311 an, an dessen Innenseite ein Innengewinde 310 ausgebildet ist, welches koaxial zum Außengewinde 320 ist und zum proximalen Ende hin offen ist, um das Trennelement 300 auf das distale Behälterende 102 aufschrauben zu können. Vom Mantelwandbereich 31 1 ausgehend erstrecken sich mehrere gleichartige, federelastische Rastarme 330 in die proximale Richtung. Die Funktion der Rastarme wird nachstehend noch näher erläutert. Die freien Enden der Rastarme 330 bilden das proximale Ende 301 des Trennelements 300. An ihren freien Enden weisen die Rastarme 330 jeweils ein sich nach innen erstreckendes Hintergreifelement 332 in Form einer Rastnase auf. Die Rastarme 330 besitzen an ihrem jeweiligen freien Ende eine Außenfläche 331. Diese Außenflächen 331 definieren den maximalen Radius Rmax des Trennelements. Sie sind radial weiter außen angeordnet als die Mantelfläche 312 des sich distal an die Rastarme anschließenden Mantelwandbereichs 31 1. Der Verschluss 400 ist in den Figuren 8 und 9 besonders gut erkennbar. Er weist ein proximales Ende 401, ein distales Ende 402, eine umlaufende Mantelwand 410 und eine Deckwand 420 auf. Der Verschluss ist von rohrförmiger Grundform, wobei er durch die Deckwand 420 in einen proximalen Abschnitt 403 und einen distalen Abschnitt 404 unterteilt wird. Sowohl der proximale Abschnitt 403 als auch der distale Abschnitt 404 sind hohl, und der Verschluss ist sowohl am proximalen Ende 401 als auch am distalen Ende 402 offen. Im proximalen Abschnitt 403 ist innenseitig in der Mantelwand 410 ein Innengewinde 430 ausgebildet. Die Mantelwand 410 erstreckt sich proximal deutlich über das Innengewinde 430 hinaus. Im distalen Abschnitt 404 sind innenseitig in der Mantelwand 410 eine Vielzahl von Mitnehmern 440 in Form axial verlaufender Rippen ausgebildet. Außenseitig weist der Verschluss eine Vielzahl von Längsrippen 450 auf, um zu verhindern, dass ein Benutzer bei der Handhabung des Verschlusses abrutscht. Bei der Herstellung wird der Behälter 100 mit einem Granulat (nicht dargestellt) befüllt. Wie insbesondere aus der Figur 4 hervorgeht, wird am distalen Behälterende 102 über der distalen Austragöffnung 104 das Trennelement 300 angebracht, wobei das Innengewinde 310 des Trennelements mit dem Außengewinde 160 des Behälters in Eingriff kommt. Auf dem Trennelement 300 ist der Verschluss 400 montiert, wobei das Innengewinde 430 des Verschlusses mit dem Außengewinde 320 des Trennelements 300 in Eingriff steht. Dabei liegt die Deckwand 420 des Verschlusses axial auf der distalen Stirnfläche des Filterbereichs 350 mit den Durchlassöffnungen 351 auf. Die Mantelwand 410 des Verschlusses überdeckt das Filterelement 300 radial vollständig, so dass der Benutzer das Filterelement 300 in diesem Zustand nicht berühren kann. Die Mantelwand 410 erstreckt sich insbesondere über die Rastarme 330 hinweg. Dabei liegt sie auf den Außenflächen 331 der Rastarme 330 auf und drückt dadurch die Rastarme 330 radial nach innen. Dadurch kommen die Hintergreifelemente 332 (hier Rastnasen) in axial formschlüssigen Eingriff mit dem Axialsicherungselement 170 (hier ein Ringwulst), so dass sich das Trennelement 300 nicht mehr in distaler Richtung vom Behälter 100 entfernen lässt.

Es resultiert der Zustand der Figuren 2-4. In dieser Form wird die Austragvorrichtung ausgeliefert. Zur Verwendung der Austragvorrichtung schraubt der Benutzer nun den Verschluss 400 ab. Der Eingriff der Hintergreifelemente 332 mit dem Axialsicherungselement 170 verhindert, dass dabei gleichzeitig auch das Trennelement 300 mitgenommen wird. Das Trennelement 300 verbleibt also auf der distalen Austragöffnung des Behälters 100 und verhindert, dass das Granulat aus dem Behälter 100 austritt. Dazu verfügen die Durchlassöffnungen 351 über einen Durchmesser, welcher kleiner als die mittlere Korngröße des Granulats ist, bestimmt durch eine Siebanalyse nach DIN EN 933-1 :2012- 03. Nun wird eine Flüssigkeit, z.B. Blut oder physiologische Kochsalzlösung, durch das Trennelement 300 hindurch in den Behälter 100 eingebracht. Dazu stehen verschiedene Möglichkeiten zur Verfügung. So kann die Flüssigkeit z.B. mit einer Spritzenkanüle durch die Durchlassöffnungen eingespritzt werden, oder die Flüssigkeit kann in den Behälter eingesaugt werden, indem das Vorschubelement 200 zurückgezogen wird.

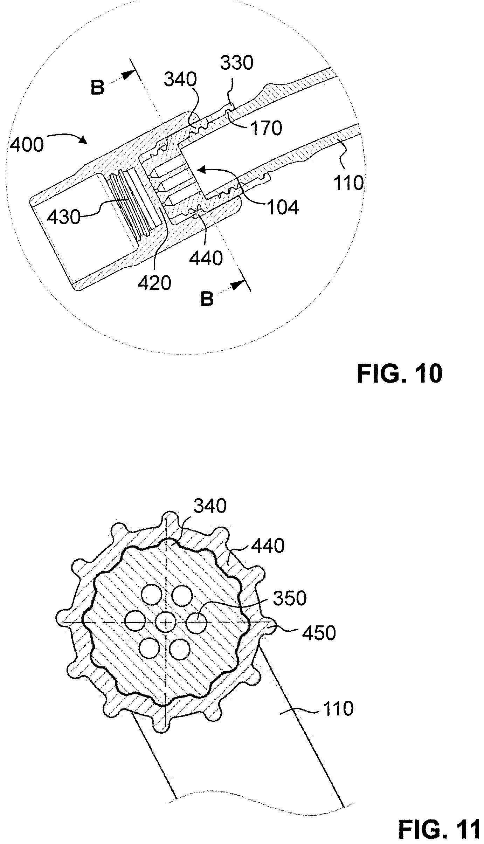

Um das so entstandene Produkt nun auszutragen, wird das Trennelement 300 vom Behälter 100 entfernt. Dazu wird der Verschluss 400 um 180 Grad um die Querrichtung gedreht und in umgekehrter Orientierung auf das Trennelement 300 aufgeschoben. Dieser Zustand ist in den Figuren 10 und 11 illustriert. Dabei kommen die Mitnehmer 440 auf der Innenseite der Mantelwand 410 des Verschlusses in Eingriff mit den Mitnahmeelementen 340 auf der Außenseite des Trennelements 300, so dass ein Formschluss zwischen Trennelement 300 und Verschluss 400 in Umfangsrichtung hergestellt wird. Dadurch kann nun das Trennelement 300 mit Hilfe des Verschlusses 400 vom Behälter 100 abgeschraubt werden, ohne dass der Benutzer das Trennelement 300 zu berühren braucht. Der axiale Eingriff zwischen den Hintergreifelementen 332 und der Axialsicherungsstruktur 170 ist dabei gelöst, da die Rastarme 330 nun nach außen ausfedern können.

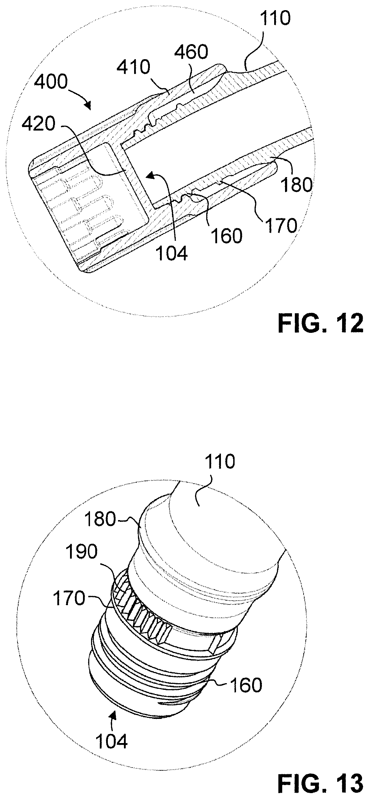

Wenn der Behälter schon mit einem gebrauchsfertigen Produkt vorbefüllt ist, kann das Trennelement 300 auch entfallen, und der Verschluss 400 kann direkt über der Austragöffnung des Behälters 100 angebracht werden. Dennoch können derselbe Behälter 100 und derselbe Verschluss 400 zum Einsatz kommen, d.h. es sind keine konstruktiven Änderungen nötig. Diese Situation ist in der Figur 12 illustriert. Die Mantelwand 410 des Verschlusses 400 erstreckt sich dabei nun in einem radialen Abstand von der Außenseite der Behälterwand 1 10, so dass sich zwischen Behälterwand 110 und Mantelwand 410 des Verschlusses 400 ein ringförmiger radialer Zwischenraum 460 ausbildet. Dabei überdeckt die Mantelwand 410 des Verschlusses insbesondere auch radial die Axialsicherungsstruktur 170. Um zu verhindern, dass Schmutz in den Zwischenraum 460 eindringt, ist die schon erwähnte Überbrückungsstruktur 180 auf der Außenseite der Behälterwand 1 10 vorgesehen. Diese erstreckt sich radial genügend weit nach außen, um den Zwischenraum 460 zu überbrücken und in die proximale Richtung abzuschließen. Dabei liegt das proximale Ende der Mantelwand 410 auf der radialen Außenseite der Überbrückungsstruktur 180 auf. Optional kann hier eine Dichtung ausgebildet sein.

Ein zweites Ausführungsbeispiel ist in den Figuren 13-16 illustriert. Gleiche oder gleich wirkende Elemente sind mit denselben Bezugszeichen wie beim ersten Ausführungsbeispiel bezeichnet. Das zweite Ausführungsbeispiel unterscheidet sich vom ersten Ausführungsbeispiel lediglich durch die Ausgestaltung des distalen Endbereichs des Behälters 100 und der Rastelemente 330 am Trennelement 300.

Der distale Endbereich des Behälters 100 ist in der Figur 13 illustriert. Zwischen der Axialsicherungsstruktur 170 (hier ein Ringwulst) und der Überbrückungsstruktur 180 (hier ebenfalls ein Ringwulst) ist an der Behälterwand 1 10 außenseitig eine Drehsicherungsstruktur 190 in Form einer Außenverzahnung ausgebildet. Aus Gründen der besseren Entformbarkeit beim Spritzgießen ist diese Außenverzahnung nicht durchgehend über den Außenumfang ausgebildet, sondern teilweise unterbrochen.

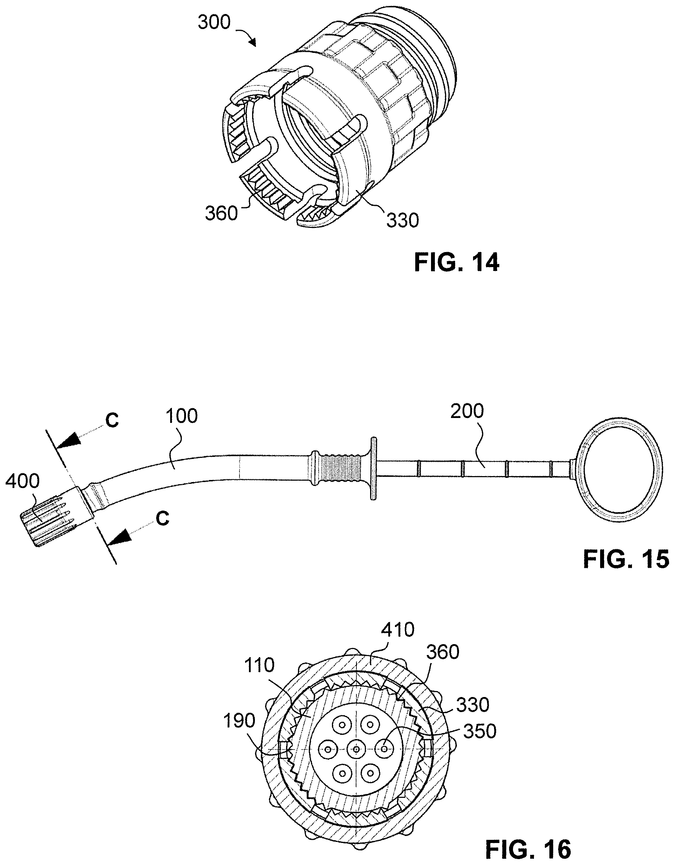

Wie aus der Figur 14 hervorgeht, ist am proximalen Ende jedes Rastarms 330 eine dazu komplementäre Innenverzahnung 360 ausgebildet. Diese bildet hier gleichzeitig auch die Hintergreifelemente für eine Axialsicherung an der Axialsicherungsstruktur 170.

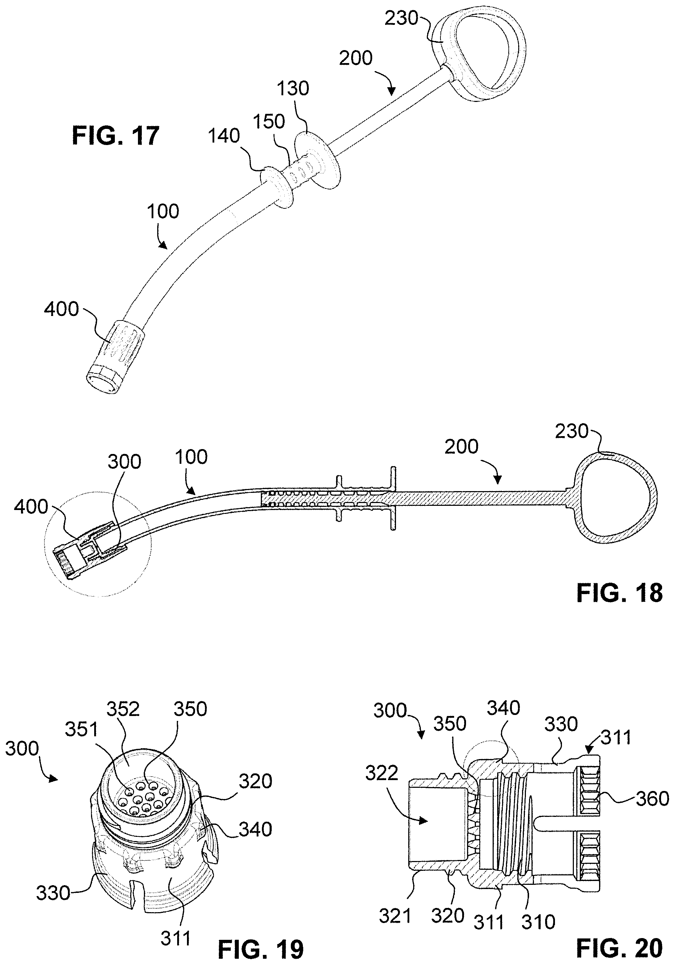

Wie in den Figuren 15 und 16 illustriert ist, wird auf diese Weise das Trennelement 300 nicht nur in axialer Richtung, sondern auch bezüglich Drehungen am Behälter 100 gesichert, wenn der Verschluss 400 auf das Trennelement 300 aufgeschraubt ist. Dabei drückt die Mantelwand 410 des Verschlusses die Rastarme 330 wiederum nach innen, so dass die Innenverzahnung 360 in formschlüssigen Eingriff sowohl mit der Drehsicherungsstruktur 190 bezüglich der Umfangsrichtung als auch mit der Axialsicherangsstruktur 170 bezüglich der distalen Richtung gelangt. Beim Abschrauben des Verschlusses ist dadurch zusätzlich sichergestellt, dass das Trennelement 300 am Behälter verbleibt. Ein drittes Ausführungsbeispiel ist in den Figuren 17 bis 24 illustriert. Gleiche oder gleich wirkende Elemente sind wiederum mit denselben Bezugszeichen wie in den vorhergehenden Ausführungsbeispielen bezeichnet.

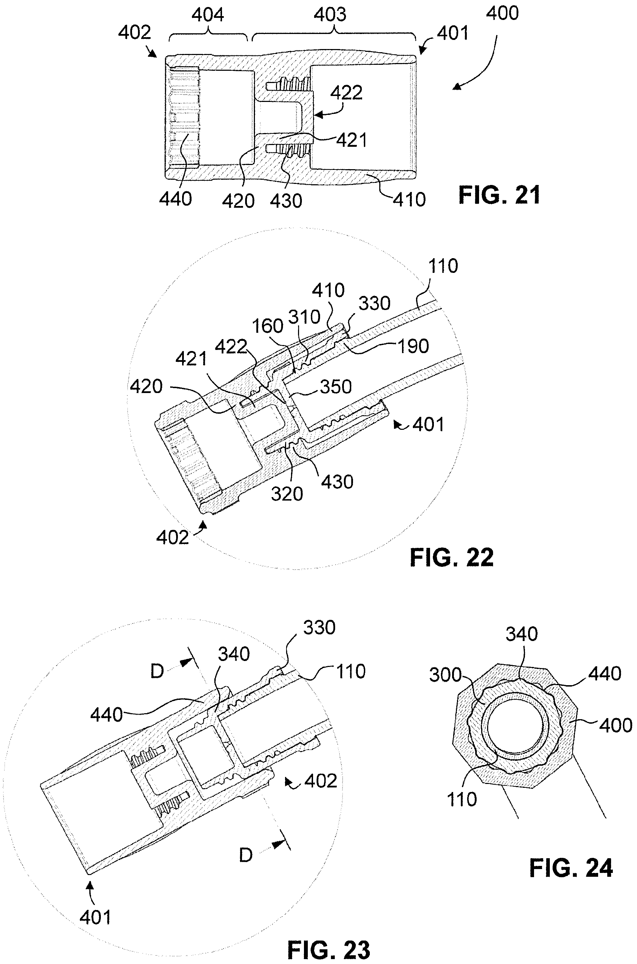

Das dritte Ausführungsbeispiel unterscheidet sich vom zweiten Ausführungsbeispiel vor allem durch die Ausgestaltung des Trennelements 300 und des Verschlusses 400. Daneben bestehen auch kleinere Unterschiede in der Gestaltung der ersten und zweiten Fingerauflagen 130, 140 und der dazwischen angeordneten Rillenstruktur 150 sowie in der Formgebung des Daumenrings 230. Das Trennelement 300 des dritten Ausführungsbeispiels ist in den Figuren 19 und 20 alleine dargestellt. Anders als im ersten und zweiten Ausführungsbeispiel erstreckt sich der Filterbereich 350 nicht bis zum distalen Ende des Trennelements 300, sondern der Filterbereich 350 ist als eine verhältnismäßig dünne Platte mit einer Vielzahl von Öffnungen 351 ausgebildet. An den Filterbereich 350 schließt sich in distaler Richtung ein Rohrstutzen 321 an, der das distale Ende des Trennelements 300 bildet. Der Rohrstutzen 321 ist hohl und definiert somit einen Hohlraum 322, der in Umfangsrichtung vom Rohrstutzen 321 und in proximaler Richtung vom Filterbereich 350 begrenzt ist. Das Außengewinde 320 ist in diesem Ausführungsbeispiel auf der Außenseite des Rohrstutzens 321 ausgebildet. Durch diese Ausgestaltung wird die Fertigung des Trennelements im Spritzgussverfahren vereinfacht. Im Bereich des Außengewindes kann die Entformung auf einfache Weise mit einem Drehkern erfolgen, während bei den ersten beiden Ausführungsbeispielen kompliziertere Maßnahmen zur Entformung erforderlich sind. Anders als in den ersten beiden Ausführungsbeispielen stehen die Mitnahmeelemente 340 des Trennelements des dritten Ausführungsbeispiels radial leicht über den sich in proximaler Richtung anschließenden Mantelwandbereich hinaus vor. Im Bereich des proximalen Endes entspricht die Ausgestaltung des Trennelements ansonsten weitgehend derjenigen des zweiten Ausführungsbeispiels. Insoweit wird auf die vorstehende Beschreibung verwiesen. In der Figur 21 ist der Verschluss des dritten Ausführungsbeispiels alleine dargestellt. Wie im ersten und zweiten Ausführungsbeispiel gliedert sich der Verschluss in einen proximalen Abschnitt 403 sowie einen distalen Abschnitt 404. Anders als im ersten und zweiten Ausführungsbeispiel ist an der umlaufend mit der Mantelwand 410 verbundenen Deckwand 420 ein Stopfen 421 ausgebildet, der an seinem distalen Ende durch einen ringförmigen Deckwandbereich mit der Mantelwand 410 verbunden ist. Der Stopfen 421 erstreckt sich im Inneren des Verschlusses 400 in die proximale Richtung. An seinem freien proximalen Ende bildet der Stopfen 421 eine distale Stirnfläche 422. Im vorliegenden Beispiel bildet die Deckwand 420 quasi durch ihren Verlauf den Stopfen 421. Das Innengewinde 430 ist in einem Bereich der Mantelwand 410 ausgebildet, welcher den Stopfen 421 radial umgibt.

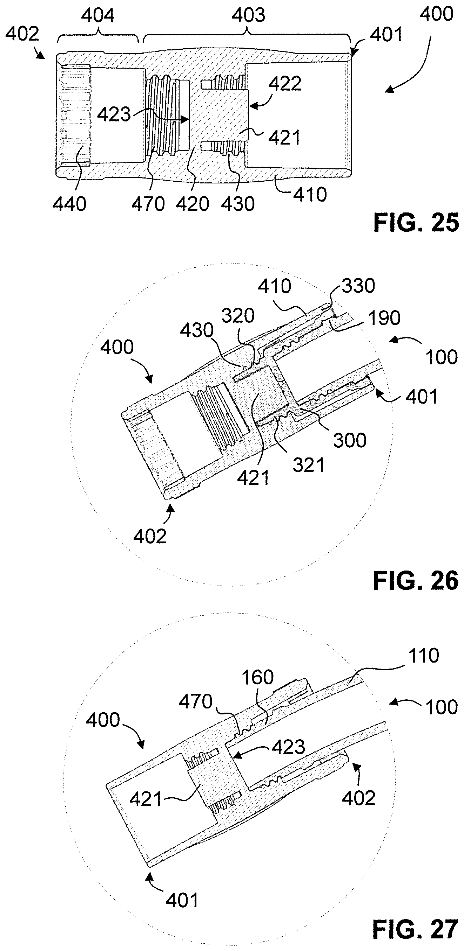

Im distalen Abschnitt 404 ist der Verschluss 400 des dritten Ausführungsbeispiels weitgehend gleich ausgebildet wie im ersten und zweiten Ausführungsbeispiel. Insbesondere weist die Mantelwand 410 des Verschlusses angrenzend an das distale Ende 402 innenseitig wiederum Mitnehmer 440 auf, die komplementär zu den Mitnahmeelementen 340 des Trennelements 300 ausgebildet sind. Wegen der leicht anderen Form der Mitnahmeelemente 340 unterscheidet sich die Formgebung der Mitnehmer 440 leicht von der Formgebung im ersten und zweiten Ausführungsbeispiel. In den Figuren 22 bis 24 ist das Zusammenwirken des Verschlusses 400 des dritten Ausführungsbeispiels mit dem Trennelement 300 illustriert. In der Figur 22 befindet sich der Verschluss 400 in der Verschließstellung. Dazu ist er mit Hilfe seines Innengewindes 430 auf das Außengewinde 320 des Trennelements 300 aufgeschraubt. Dabei überdeckt die Mantelwand 410 im proximalen Abschnitt wiederum die Rastarme 330 des Trennelements 300, wie dies vorstehend schon für das erste und zweite Ausführungsbeispiel erläutert wurde. Der Stopfen 421 ragt in der proximalen Richtung in den Hohlraum 352 hinein, und zwar so weit, dass die proximale Stirnfläche 422 unmittelbar auf der distalen Seite des Filterbereichs 350 aufliegt. Auf diese Weise wird vermieden, dass besonders feine Partikel eines im Behälter 100 aufgenommenen Granulats, die durch den Filterbereich 350 hindurchtreten können, sich im Hohlraum 352 ansammeln können. Um das im Behälter 100 aufgenommene Granulat mit einer Flüssigkeit zu benetzen, wird der Verschluss 400 abgeschraubt, und Flüssigkeit wird in den Behälter 100 aufgesaugt, wie dies in größerem Detail im Zusammenhang mit dem ersten und zweiten Ausführungsbeispiel erläutert wurde. Um anschließend das Trennelement 300 vom Behälter 100 zu entfernen, wird der Verschluss 400 wiederum in umgekehrter Orientierung auf das Trennelement 300 aufgesteckt, und das Trennelement 300 wird mit Hilfe des Verschlusses vom Behälter 100 abgeschraubt. Diese Situation ist in den Figuren 23 und 24 illustriert, wobei in der Figur 24 besonders gut das Zusammenwirken der Mitnahmeelemente 340 mit den Mitnehmern 440 erkennbar ist. Ein viertes Ausführungsbeispiel ist in den Figuren 25 bis 28 illustriert. Dieses Ausführungsbeispiel unterscheidet sich vom dritten Ausführungsbeispiel lediglich durch die Gestaltung des Verschlusses, der in der Figur 25 alleine dargestellt ist. In diesem Ausführungsbeispiel weist der Verschluss zwischen dem Stopfen 421 und dem distalen Ende 402 des Verschlusses ein zweites Innengewinde 470 auf, welches gleich dimensioniert ist wie das erste Innengewinde 430, aber anders als das erste Innengewinde 430 zum distalen Ende 402 hin offen ist. Der Stopfen 421 ist in diesem Ausführungsbeispiel als massiver Zylinder ausgebildet, so dass die Deckwand 420 auf der distalen Rückseite des Stopfens 421 eine ebene distale Stirnfläche 423 bildet, die über den gesamten lichten Querschnitt des Stopfens eben ist. Das zweite Innengewinde 470 schließt sich an ebene distale Stirnfläche 423 in die distale Richtung an.

Ein solcher Verschluss ist vor allem dann wertvoll, wenn in den gleichen Behälter 100 wahlweise ein zu benetzendes Granulat oder ein gebrauchsfertiges Produkt aufgenommen werden soll und der Behälter mit dem gleichen Verschluss verschlossen werden soll. Dies wird anhand der Figuren 26 bis 28 näher erläutert.

In der Figur 26 ist die Situation dargestellt, in der der Verschluss auf ein Trennelement 300 aufgeschraubt ist, wobei das Trennelement 300 genauso ausgestaltet ist wie im dritten Ausführungsbeispiel. Insbesondere ragt der Stopfen 421 in den Hohlraum hinein, der durch den Rohrstutzen 321 des Trennelements 300 begrenzt wird.

In der Figur 27 ist die Situation dargestellt, in der der Verschluss 400 in umgekehrter Orientierung unmittelbar auf das distale Ende des Behälters 100 aufgeschraubt ist. Dabei greift das zweite Innengewinde 470 in das Außengewinde 160 am distalen Behälterende ein. Die distale Stirnfläche 423 auf der Rückseite des Stopfens 421 liegt flach auf dem distalen Behälterende auf und überdeckt dieses dadurch. Dadurch ragt kein Element des Verschlusses 400 in das Innere des Behälters 100 hinein. Falls umgekehrt der Verschluss 400 in der Orientierung der Figur 26 auf das distale Behälterende aufgeschraubt würde, würde der Stopfen 421 ins Innere des Behälters 100 hineinragen und Material, welches sich in diesem Bereich befindet, verdrängen. Durch die vorgeschlagene Gestaltung mit einem zweiten Innengewinde wird dies vermieden. Auf diese Weise kann derselbe Verschluss wahlweise verwendet werden, um auf ein Trennelement aufgeschraubt zu werden, oder er kann verwendet werden, um direkt auf das Behälterende aufgeschraubt zu werden.

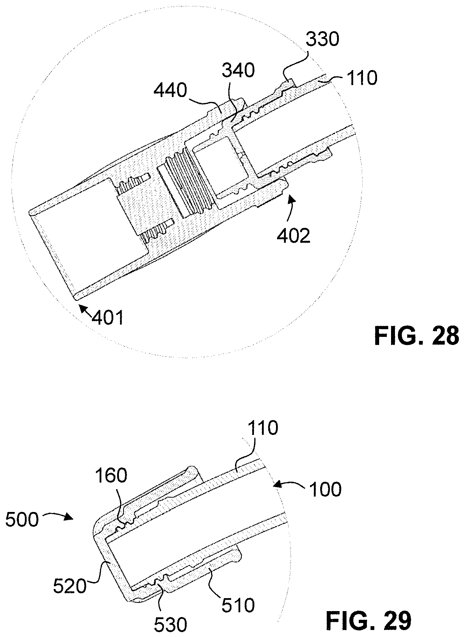

Der Vollständigkeit halber ist in der Figur 28 die Situation illustriert, in der der Verschluss in der umgekehrten Orientierung auf das Trennelement aufgeschoben wurde, um dieses vom Behälter abzuschrauben. Insofern weist der Verschluss des vierten Ausführungsbeispiels sämtliche Funktionalitäten des Verschlusses der anderen Ausführungsbeispiele auf.

Ein fünftes Ausführungsbeispiel ist in der Figur 29 illustriert. In diesem Ausführungsbeispiel wird für den Fall, dass der Behälter direkt verschlossen werden soll, ein separater Kappenverschluss 500 bereitgestellt, welche in üblicher Weise eine Mantelwand 510 mit Innengewinde 530 sowie eine Deckwand 520 aufweist.

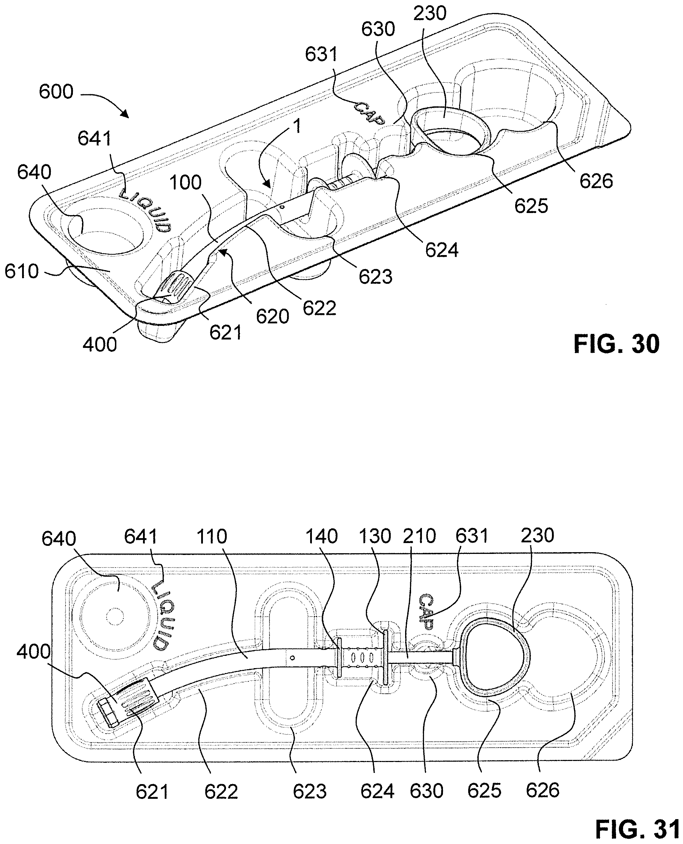

In den Figuren 30 bis 35 ist die Kombination einer Austragvorrichtung 1 der vorstehend beschriebenen Art mit einer Verpackung 600 illustriert. Die Verpackung 600 ist als Blisterverpackung ausgebildet. Sie weist einen Träger 610 auf, der in üblicher Weise als thermogeformtes Folienformteil ausgebildet ist. Der Träger 610 definiert eine horizontale Oberseite. Der Träger 610 weist eine nach oben offene Aufnahmevertiefung 620 zur liegenden Aufnahme der Austragvorrichtung 1 auf. Die Aufnahmevertiefung gliedert sich in mehrere Abschnitte, nämlich in einen ersten Abschnitt 621, der das distale Ende der Austragvorrichtung 1 mit dem Verschluss 400 aufnimmt, einen zweiten Abschnitt 622, der den gekrümmten Abschnitt des Behälters 100 aufnimmt, einen dritten Abschnitt 623, in dem die Aufnahmevertiefung zur erleichterten Entnahme der Austragvorrichtung 1 stark aufgeweitet ist, einen vierten Abschnitt 624 zur Aufnahme der beiden Fingerauflagen und des dazwischen liegenden Bereichs, sowie einen fünften und sechsten Abschnitt 625, 626, die jeweils zur Aufnahme des Daumenrings 230 ausgebildet sind. Die Austragvorrichtung 1 ist in die Aufnahmevertiefung 620 eingelegt, wobei in der Situation der Figuren 30 und 31 der Daumenring 230 im fünften Abschnitt 625 der Aufnahmevertiefung 620 zu liegen kommt. Der Behälter ist hier etwa zur Hälfte mit einem zu benetzenden Granulat gefüllt und mit einem Trennelement 300 und einem Verschluss 400 gemäß dem dritten Ausführungsbeispiel verschlossen. Alternativ könnte auch ein Verschluss 400 nach einem der anderen Ausführungsbeispiele vorgesehen sein. Das Vorschubelement 200 ist etwa zur Hälfte in den Behälter 100 eingeschoben. Nach der Entnahme der Austragvorrichtung 1 und dem Entfernen des Verschlusses 400 ist es daher möglich, durch das Trennelement 300 hindurch eine Flüssigkeit in den Behälter 100 aufzusaugen, indem das Vorschubelement 200 mittels des Daumenrings 230 zurückgezogen wird.

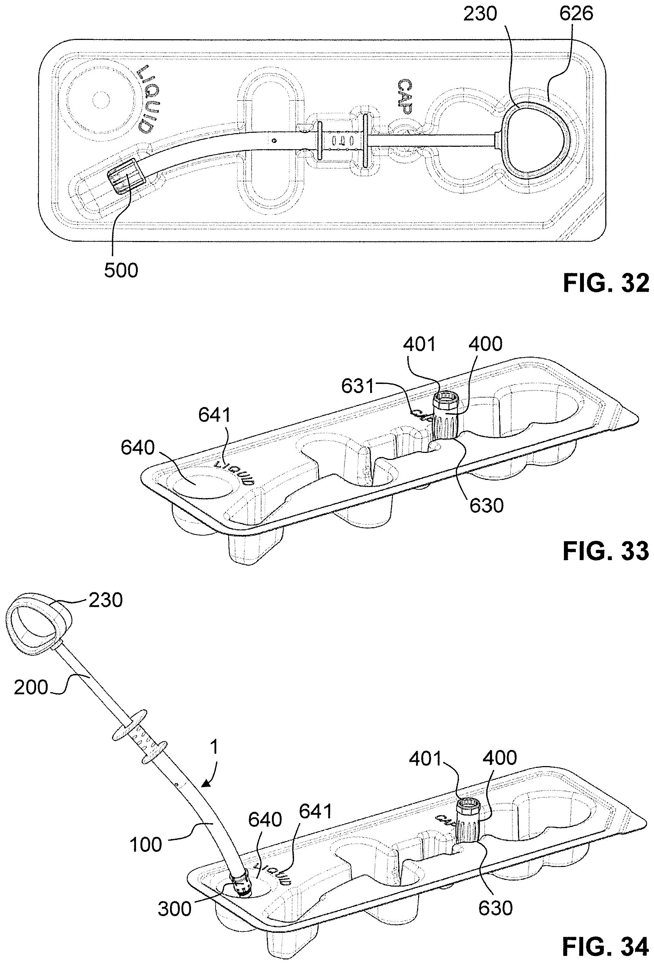

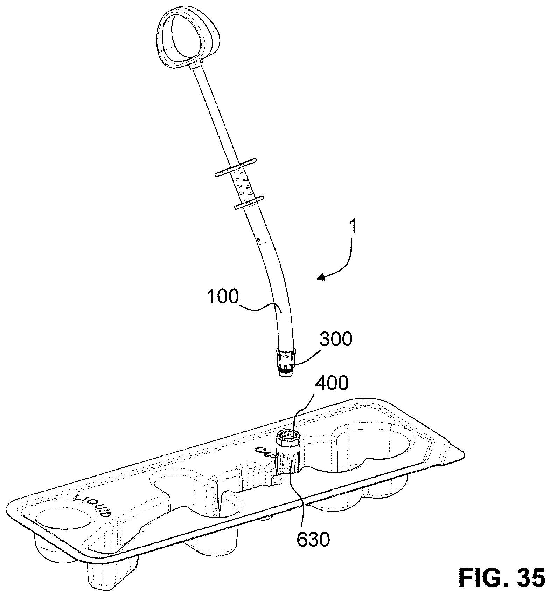

In der Situation der Figur 32 ist der Behälter dagegen vollständig mit einem austragfertigen Produkt (z.B. einem fertigen Knochenersatzmaterial) gefüllt, und das Vorschubelement 200 ist dementsprechend vollständig zurückgezogen, sodass der Daumenring 230 im Abschnitt 626 der Aufnahmevertiefung 620 zu liegen kommt. Der Behälter ist hier direkt (ohne dazwischen liegendes Trennelement) mit dem Verschluss 500 des fünften Ausführungsbeispiels (gemäß der Figur 29) verschlossen, könnte aber auch mit dem Verschluss 400 der anderen Ausführungsbeispiele verschlossen sein. Zwischen den Abschnitten 624 und 625 befindet sich ein weiterer Abschnitt der Aufnahmevertiefung 620, welcher eine Verschlusshaltevertiefung 630 bildet. Die Verschlusshaltevertiefung 630 ist komplementär zum Verschluss 400 dimensioniert. Dadurch lässt sich der Verschluss 400 derart in die Verschlusshaltevertiefung einstecken, dass das distale Verschlussende nach oben weist. Diese Situation ist in der Figur 33 illustriert. Neben der Verschlusshaltevertiefung 630 ist am Träger eine entsprechende Markierung 631 ("CAP") ausgebildet. Zusätzlich ist im Träger 610 eine nach oben offene, separate Fluidreservoirvertiefung 640 ausgebildet, um eine Flüssigkeit wie Kochsalzlösung oder Blut aufzunehmen. Die Fluidreservoirvertiefung 640 ist mit einer entsprechenden Markierung 641 ("LIQUID") markiert. In den Figuren 34 und 35 ist die Verwendung der Verpackung 600 illustriert. Der Benutzer entnimmt die mit Granulat vorbefüllte Austragvorrichtung 1 aus dem Träger 610. Dazu kann es erforderlich sein, dass er zunächst eine nicht dargestellte Deckschicht vom Träger 610 entfernt, z.B. eine Kunststofffolie, die im Auslieferungszustand die gesamte Oberseite des Trägers 610 überdeckt, um den Inhalt der Verpackung zu schützen und steril zu halten. Der Benutzer schraubt sodann den Verschluss 400 vom Trennelement 300 ab und setzt den Verschluss mit dem distalen Ende 401 nach oben in die Verschlusshaltevertiefung 630 ein (siehe Figur 34). Nun gibt der Benutzer eine Flüssigkeit, z.B. Kochsalzlösung oder Blut, in die Fluidreservoirvertiefung 640. Der Benutzer ergreift anschließend die Austragvorrichtung 1, taucht das Trennelement 300 in die Flüssigkeit in der Fluidreservoirvertiefung 640 hinein und saugt diese in den Behälter 100 auf, indem er das Vorschubelement 200 mit Hilfe des Daumenrings 230 in die proximale Richtung P zurückzieht. Dadurch wird das Granulat im Behälter mit der Flüssigkeit benetzt.

Nach diesem Vorgang ist das Trennelement 300 äußerlich mit der Flüssigkeit kontaminiert und sollte nicht mehr berührt werden. Um das Trennelement 300 zu entfernen, ohne es zu berühren, steckt der Benutzer die Austragvorrichtung 1 mit dem Trennelement 300 in den Verschluss 400 hinein und entnimmt auf diese Weise den Verschluss 400 aus der Verschlusshaltevertiefung 630 (siehe Figur 35). Nun kann der Benutzer das Trennelement 300 mit Hilfe des Verschlusses 400 vom Behälter 100 abschrauben, ohne das Trennelement 300 zu berühren. Anschließend kann der Benutzer das benetzte Granulat austragen. Insgesamt kommt der Benutzer auf diese Weise zu keinem Zeitpunkt mit dem kontaminierten Trennelement 300 in Berührung. Auch besteht keine Gefahr, dass der Benutzer versehentlich mit dem kontaminierten Trennelement 300 in Berührung kommt.

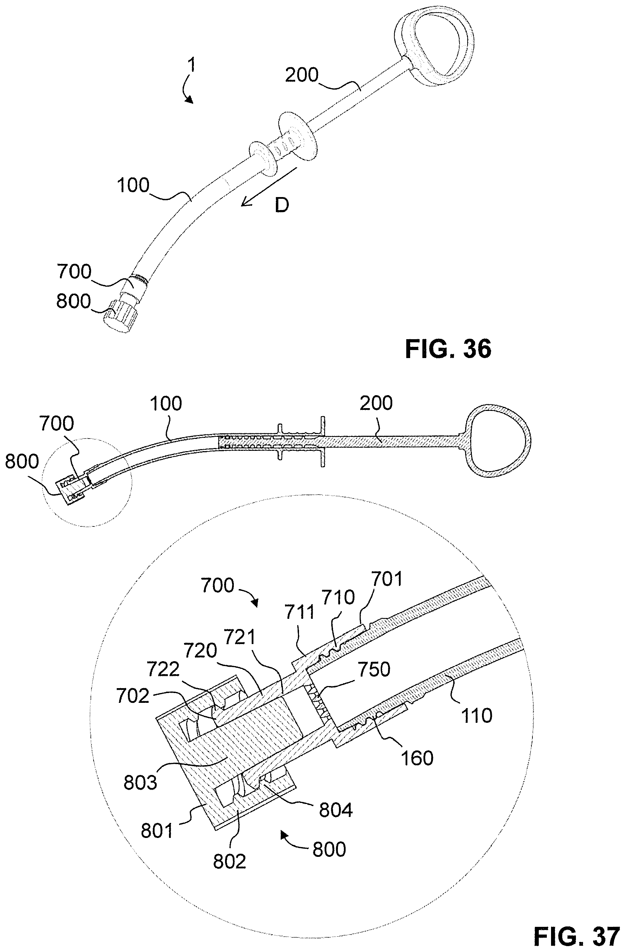

In den Figuren 36 bis 38 ist ein sechstes Ausführungsbeispiel einer Austragvorrichtung 1 illustriert. Bei diesem Ausführungsbeispiel ist ein Trennelement 700 vorhanden, welches besonders dazu ausgestaltet ist, mit einer handelsüblichen Spritze verbunden zu werden, um Flüssigkeit aus einer solchen Spritze in den Behälter 100 aufzunehmen.

Die Austragvorrichtung 1 umfasst wiederum einen Behälter 100, in dem ein Vorschubelement 200 verschiebbar angeordnet ist. Über dem distalen Ende des Behälters 100 ist das erwähnte Trennelement 700 angebracht. Das Trennelement 700 weist einen Filterbereich 750 mit einer Vielzahl von Filteröffnungen auf, wobei der Filterbereich 750 auf dem distalen Behälterende aufliegt. Proximal vom Filterbereich 750 ist eine umlaufende Mantelwand 71 1 mit einem Innengewinde 710 ausgebildet. Das Innengewinde 710 steht mit einem Außengewinde 160 am Behälter im Bereich von dessen distalem Ende in Eingriff. Distal vom Filterbereich 750 ist ein Anschlussbereich 720 ausgebildet, welcher einen weiblichen Luerkonus 721 bildet. Am freien distalen Ende des Anschlussbereichs 720 sind außenseitig zwei Eingriffselemente 722 in Form von zwei kurzen Segmenten eines Außengewindes ausgebildet. Ein Verschluss 800 ist auf das Trennelement 700 aufgeschraubt. Der Verschluss 800 weist eine Deckwand 801 sowie eine umlaufende Mantelwand 802 auf. Von der Deckwand ausgehend erstreckt sich ein Stopfen 803 in die proximale Richtung. Der Stopfen bildet einen männlichen Luerkonus. An der Mantelwand 802 ist innenseitig eine Innengewinde 804 ausgebildet, welches mit den Eingriffselementen 722 des Trennelements 700 in Eingriff steht.

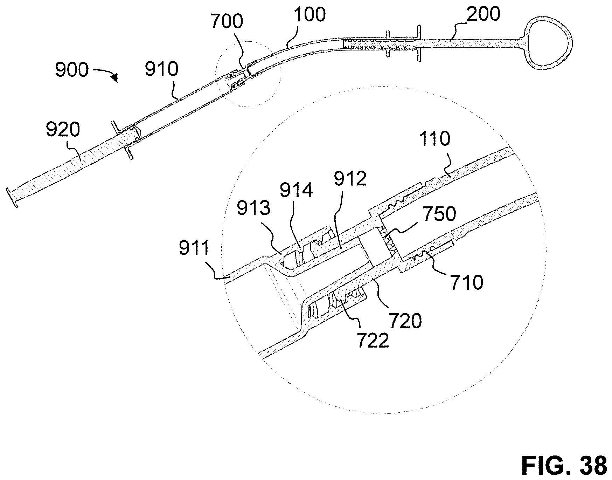

Um ein im Behälter 100 aufgenommenes Granulat mit Flüssigkeit zu benetzen, wird zunächst der Verschluss 800 vom Trennelement 700 entfernt. Anschließend wird eine handelsübliche Spitze 900 mit einem Fluidreservoir 910, in dem ein Kolben 920 verschiebbar ist, mit dem Trennelement 700 verbunden (siehe Figur 38). Das Fluidreservoir 910 der Spritze 900 weist eine Mantelwand 91 1 auf, die eine Begrenzungswand für das Fluidreservoir 910 bildet und an deren distales Ende sich ein Auslassbereich 912 anschließt. Der Auslassbereich 912 bildet außenseitig einen männlichen Luerkonus. Er ist radial von einem starr mit der Mantelwand 911 verbundenen Befestigungsring 913 umgeben, an dem ein Innengewinde 914 ausgebildet ist. Das Fluidreservoir 910 wird mit dem Trennelement 700 verbunden, indem der Auslassbereich 912 in den Anschlussbereich 720 des Trennelements 700 eingeschoben wird und anschließend durch eine Relativdrehung das Innengewinde 914 im Befestigungsring 913 mit den Eingriffselementen 722 in Eingriff gebracht wird. Dadurch wird die Spritze 900 mit dem Trennelement 700 verriegelt. Nun kann das Vorschubelement 200 der Austragvorrichtung zurückgezogen werden, und/oder der Kolben 920 in der Spritze 900 kann vorgeschoben werden, um Flüssigkeit aus dem Reservoir 910 der Spritze in den Behälter 100 der Austragvorrichtung zu überführen. Dadurch wird das im Behälter 100 aufgenommene Granulat mit der Flüssigkeit benetzt.

Um das so erhaltene Produkt auszutragen, werden die Spritze 900 und das Trennelement 700 vom Behälter 100 entfernt, und das Produkt kann durch das nun offene distale Ende des Behälters 100 hindurch ausgetragen werden.

**BEZUGSZEICHENLISTE**

| | |
|---|---|
| 1 Austragvorrichtung | 321 Rohr stutzen |
| | 322 Hohlraum |
| 100 Behälter | 330 Rastarm |
| 101 proximales Behälterende | 331 Außenfläche |
| 102 distales Behälterende | 332 Hintergreifelement |
| 103 Einführöffnung | 340 Mitnahmeelement |
| 104 Austragöffnung | 350 Filterbereich |
| 110 Behälterwand | 351 Duchlassöffnung |
| 120 Behälterinnenraum | 360 Eingriffsstruktur |
| 130 erste Fingerauflage | |
| 140 zweite Fingerauflage | 400 Verschluss |
| 150 Rillenstruktur | 401 proximales Ende |
| 160 Aussengewinde | 402 distales Ende |
| 170 Axialsicherungsstruktur | 403 proximaler Abschnitt |
| 180 Überbrückungsstruktur | 404 distaler Abschnitt |
| 190 Drehsicherungsstruktur | 410 Mantelwand |
| | 420 Deckwand |
| 200 Vorschubelement | 421 Stopfen |
| 210 Kolbenstange | 422 proximale Stirnfläche |
| 211 Führungswulst | 423 distale Stirnfläche |
| 212 distaler Bereich | 430 Innengewinde |
| 220 Kolben | 440 Mitnehmer |
| 230 Daumenring | 450 Längsrippe |
| | 460 Zwischenraum |
| 300 Trennelement | 470 zweites Innengewinde |
| 301 proximales Ende | |
| 302 distales Ende | 500 Verschluss |
| 310 Innengewinde | 510 Mantelwand |
| 311 Mantelwandbereich | 520 Deckwand |
| 312 Außenfläche | 530 Innengewinde |
| 320 Außengewinde | |
| 600 Verpackung | 801 Deckwand |
| 610 Träger | 802 Mantelwand |
| 620 Aufnahmevertiefung | 803 Stopfen |
| 621-626 Vertiefungsbereiche | 804 Innengewinde |
| 630 Verschlusshaltevertiefung | |
| 631 Markierung | 900 Spritze |
| 640 Fluidreservoirvertiefung | 910 Reservoir |
| 641 Markierung | 911 Mantelwand |
| 700 Trennelement | 912 Auslassbereich |
| 701 proximales Ende | 913 Befestigungsring |
| 702 distales Ende | 914 Innengewinde |
| 710 Innengewinde | 920 Kolben |
| 711 Mantelwand | |
| 720 Anschlussbereich | L zentrale Längsachse |
| 721 weiblicher Luerkonus | D distale Richtung |
| 722 Eingriffselement | P proximale Richtung |
| 750 Filterbereich | R-max maximaler Radius |
| | α Krümmungswinkel |
| 800 Verschluss | |

## Patentansprüche

1. Trennelement (300) für eine medizinische Austragvorrichtung zum Austragen eines Produkts mit einem proximalen Ende (301) und einem distalen Ende (302), aufweisend:
einen Filterbereich (350), der dazu ausgebildet ist, den Durchtritt eines Granulats entlang einer axialen Richtung (L) zwischen dem proximalen Ende (301) und dem distalen Ende (302) zu verhindern, aber den Durchtritt von Flüssigkeit entlang der axialen Richtung (L) zu erlauben; und
ein zum proximalen Ende (301) hin offenes Innengewinde (310), welches um die axiale Richtung (L) verläuft;
**gekennzeichnet durch** mindestens einen außenumfänglich angeordneten, federelastischen Rastarm (330), welcher im Bereich des proximalen Endes (301) ausgebildet ist und sich mit seinem freien Ende zum proximalen Ende (301) hin erstreckt, und dass das Trennelement (300) ein oder mehrere Mitnahmeelemente (340) aufweist, die außenseitig auf dem Trennelement (300) ausgebildet sind, um in Umfangsrichtung einen formschlüssigen Eingriff des Trennelements (300) mit einem Mitnehmer eines Verschlusses zu ermöglichen.

2. Trennelement (300) nach Anspruch 1, wobei der mindestens eine Rastarm (330) im Bereich seines freien Endes innenseitig ein Hintergreifelement (332) aufweist, insbesondere in Form einer sich radial nach innen erstreckenden Rastnase, und/oder
wobei der mindestens eine Rastarm (330) im Bereich seines freien Endes innenseitig eine Eingriffsstruktur (360) aufweist, die dazu ausgebildet ist, mit einer Drehsicherungsstruktur (190) an einem Behälter (100) zusammenzuwirken, wobei die innenseitige Eingriffsstruktur (360) insbesondere in Form einer innenseitigen Verzahnung, die um die axiale Richtung (L) verläuft, ausgebildet ist.

3. Trennelement (300) nach einem der vorhergehenden Ansprüche, wobei der mindestens eine Rastarm (330) derart außenumfänglich am Trennelement (300) ausgebildet ist, dass eine Außenfläche (331) des mindestens einen Rastarms zu einer distal vom Rastarm gelegenen Außenfläche (31 1) des Trennelements (300) bündig oder radial nach außen versetzt angeordnet ist, und
wobei sich der mindestens eine Rastarm (330) vorzugsweise bis in einen proximal vom Innengewinde (310) gelegenen Bereich in die axiale Richtung (L) erstreckt.

4. Trennelement (300) nach einem der vorhergehenden Ansprüche, welches ein zum distalen Ende (302) hin offenes Außengewinde (320) aufweist, welches um die axiale Richtung (L) verläuft, und wobei
das Außengewinde (320) vorzugsweise an einem Rohrstutzen (321) ausgebildet ist, welcher innenseitig einen Hohlraum (352) definiert, wobei der Hohlraum (352) in proximaler Richtung vom Filterbereich (350) begrenzt ist.

5. Austragvorrichtung, aufweisend:
ein Trennelement (300) nach einem der vorhergehenden Ansprüche; und
einen Behälter (100) zur Aufnahme eines Produkts, mit einer umlaufenden Behälterwand (110), einem proximalen Behälterende (101), einem distalen Behälterende (102) und einer Austragöffnung (104) am distalen Behälterende (102),
wobei an der Behälterwand (1 10) im Bereich des distalen Behälterendes (102) ein Außengewinde (160) ausgebildet ist, welches um die axiale Richtung (L) verläuft, und wobei das Außengewinde (160) des Behälters (100) mit dem Innengewinde (310) des Trennelements (300) in Eingriff bringbar ist, um das Trennelement (300) am distalen Behälterende (102) anzubringen.

6. Austragvorrichtung nach Anspruch 5,
wobei proximal vom Außengewinde (160) des Behälters (100) außenseitig an der Behälterwand (110) eine Axialsicherungsstruktur (170) ausgebildet ist,
wobei der mindestens eine Rastarm (330) des Trennelements (300) mit der Axialsicherungsstruktur (170) derart in Eingriff bringbar ist, dass eine proximale Bewegung des Trennelements (300) relativ zum Behälter (100) behindert wird, und durch eine radiale Auslenkung des Rastarms (330) nach außen außer Eingriff bringbar ist.

7. Austragvorrichtung nach einem der Ansprüche 5-6,
wobei proximal vom Außengewinde (160) des Behälters (100) außenseitig an der Behälterwand (1 10) eine Drehsicherungsstruktur (190) ausgebildet ist,
wobei innenseitig am mindestens einen Rastarm (330) des Trennelements (300) eine komplementäre Eingriffsstruktur (360) ausgebildet ist, die derart mit der Drehsicherungsstruktur (190) in Eingriff bringbar ist, dass sie eine Drehung des Trennelements (300) relativ zum Behälter (100) behindert, und die durch eine radiale Auslenkung des Rastarms (330) nach außen außer Eingriff bringbar ist.

8. Austragvorrichtung nach einem der Ansprüche 5-7, aufweisend einen Verschluss (400) mit einer umlaufenden Mantelwand (410), einem proximalen Verschlussende (401), einem distalen Verschlussende (402) und einer Deckwand (420), wobei die Deckwand (420) des Verschlusses (400) in einer Verschließposition das distale Ende (302) des Trennelements (300) axial überdeckt, und
wobei die Mantelwand (410) in der Verschließposition den mindestens einen Rastarm (330) radial derart überdeckt, dass die Mantelwand (410) eine radiale Auslenkung des Rastarms (330) behindert, und
wobei sich die Mantelwand (410) des Verschlusses (400) in der Verschließposition in proximaler Richtung vorzugsweise mindestens bis zum proximalen Ende (301) des Trennelements (300) erstreckt, so dass der Verschluss (400) das Trennelement (300) in der Verschließposition vollständig überdeckt.

9. Austragvorrichtung nach Anspruch 8,
wobei in der Mantelwand (410) des Verschlusses (400) ein zum proximalen Verschlussende (401) hin offenes Innengewinde (430) ausgebildet ist, welches um die axiale Richtung (L) verläuft,
wobei das Trennelement (300) ein zum distalen Ende (302) hin offenes Außengewinde (320) aufweist, welches um die axiale Richtung (L) verläuft, und
wobei in der Verschließposition das Außengewinde (320) des Trennelements (300) mit dem Innengewinde (430) des Verschlusses (400) in Eingriff steht.

10. Austragvorrichtung nach Anspruch 9,
wobei das Außengewinde (320) des Trennelements (300) und das Außengewinde (160) des Behälters (100) gleich dimensioniert sind, so dass der Verschluss (400) mit seinem Innengewinde (430) wahlweise auf das Trennelement (300) oder direkt auf den Behälter (100) aufschraubbar ist, und/oder
wobei das Außengewinde (320) des Trennelements (300) an einem Rohrstutzen (321) ausgebildet ist, welcher innenseitig einen Hohlraum (352) definiert, wobei der Hohlraum (352) in proximaler Richtung vom Filterbereich (350) begrenzt ist, und
wobei der Verschluss einen Stopfen (421) aufweist, der an der Deckwand (420) des Verschlusses (400) ausgebildet ist, wobei sich der Stopfen (421) im Inneren des Verschlusses (400) in Richtung des proximalen Verschlussendes (401) erstreckt und sich in der Verschließstellung in den Hohlraum (352) hinein erstreckt, vorzugsweise derart, dass eine proximale Stirnfläche (422) des Stopfens (421) in der Verschließstellung auf dem Filterbereich (350) aufliegt.

11. Austragvorrichtung nach Anspruch 10,
wobei die Deckwand (420) eine distale Stirnfläche (423) definiert,
wobei der Verschluss (400) zwischen der distalen Stirnfläche (423) und dem distalen Verschlussende (402) ein zweites Innengewinde (470) aufweist, welches zum distalen Verschlussende (402) hin offen ist, um die axiale Richtung (L) verläuft und komplementär zum Außengewinde (160) am distalen Behälterende (102) ausgebildet ist, und
wobei der Verschluss (400) mit dem zweiten Innengewinde (470) in einer gegenüber der Verschließposition umgekehrten Orientierung auf das Außengewinde (160) am distalen Behälterende (102) aufschraubbar ist, so dass die distale Stirnfläche (423) das distale Behälterende (102) überdeckt.

12. Austragvorrichtung nach einem der Ansprüche 8-11,
wobei die Mantelwand (410) des Verschlusses (400) distal von der Deckwand (420) innenseitig einen oder mehrere Mitnehmer (440) aufweist, wobei das Trennelement außenseitig ein oder mehrere Mitnahmeelemente (340) aufweist, und
wobei die Mitnehmer (440) mit den Mitnahmeelementen (340) des Trennelements (300) in Eingriff bringbar sind, indem der Verschluss (400) in einer gegenüber der Verschließposition umgekehrten Orientierung entlang der axialen Richtung (L) mit dem Trennelement (300) verbunden, insbesondere auf dieses aufgeschoben, wird, so dass das Trennelement (300) mit Hilfe des Verschlusses (400) vom Behälter (100) abschraubbar ist.

13. Kombination einer Austragvorrichtung nach einem der Ansprüche 5 bis 12 mit einer Verpackung (600), die einen Träger (610) umfasst, der eine horizontale Oberseite definiert, wobei in dem Träger (610) eine nach oben offene Aufnahmevertiefung (620) zur liegenden Aufnahme der Austragvorrichtung ausgebildet ist,
wobei im Träger (610) eine Verschlusshaltevertiefung (630) ausgebildet ist, die komplementär zum Verschluss (400) dimensioniert ist, wobei der Verschluss (400) derart in die Verschlusshaltevertiefung (630) einsteckbar ist, dass das distale Verschlussende (401) nach oben weist,
wobei die Verschlusshaltevertiefung (630) separat von der Aufnahmevertiefung (620) ausgebildet ist oder als Bereich der Aufnahmevertiefung (620) ausgebildet ist.

14. Kombination nach Anspruch 13,
wobei die Austragvorrichtung ein Vorschubelement (200) aufweist, um das im Behälter aufgenommene Produkt nach Entfernung des Trennelements durch die Austragöffnung aus dem Behälter auszutragen,
wobei das Vorschubelement (200) einen Daumenring (230) aufweist, und wobei die Aufnahmevertiefung (620) einen ersten Vertiefungsbereich (625) für die Aufnahme des Daumenrings (230) aufweist, wobei der Daumenring (230) im ersten Vertiefungsbereich (625) zu liegen kommt, wenn sich das Vorschubelement relativ zum Behälter (100) in einer ersten Vorschubstellung befindet, und
wobei die Aufnahmevertiefung (620) einen zweiten Vertiefungsbereich (626) für die Aufnahme des Daumenrings (230) aufweist, wobei der Daumenring (230) im zweiten Vertiefungsbereich (625) zu liegen kommt, wenn sich das Vorschubelement in einer zweiten Vorschubstellung befindet, die sich von der ersten Vorschubstellung unterscheidet.

15. Kombination nach einem der Ansprüche 13-14, wobei in dem Träger (610) eine nach oben offene, separate Fluidreservoirvertiefung (640) ausgebildet ist, die es ermöglicht, ein Fluid aufzunehmen und dieses mit der Austragvorrichtung aufzusaugen, nachdem der Verschluss (400) von der Austragvorrichtung entfernt wurde.

## Claims

1. A separator element (300) for a medical discharge device for discharging a product with a proximal end (301) and a distal end (302), said separator element (300) comprising:
a filter region (350) which is configured to prevent the passage of a granulate along an axial direction (L) between the proximal end (301) and the distal end (302) but to permit the passage of liquid along the axial direction (L); and
an internal thread (310) which is open toward the proximal end (301) and which extends around the axial direction (L),
**characterized by** at least one resilient locking arm (330) which is arranged on the outer periphery, which is formed in the region of the proximal end (301) and which extends with its free end toward the proximal end (301), and in that the separator element (300) has one or more driver elements (340) which are formed at the outer side on the separator element (300) in order to permit a form-fitting engagement of the separator element (300) with a driver of a closure in the peripheral direction.

2. A separator element (300) in accordance with claim 1, wherein the at least one locking arm (330) has a rear-engagement element (332), in particular in the form of a radially inwardly extending locking nose, in the region of its free end at the inner side, and/or
wherein the at least one locking arm (330) has, in the region of its free end at the inner side, an engagement structure (360) which is configured to cooperate with a rotational locking structure (190) at a container (100),
wherein the inner-side engagement structure (360) is in particular configured in the form of an internal toothing which extends around the axial direction (L).

3. A separator element (300) in accordance with one of the preceding claims, wherein the least one locking arm (330) is formed on the outer periphery at the separator element (300) such that an outer surface (331) of the at least one locking arm is arranged flush with an outer surface (311) of the separator element (300) located distally from the locking arm or is arranged offset radially outwardly, and
wherein the at least one locking arm (330) preferably extends as far as a region located proximally from the internal thread (310) in the axial direction (L).

4. A separator element (300) in accordance with any one of the preceding claims which has an external thread (320) which is open toward the distal end (302) and which extends around the axial direction (L), and wherein the external thread (320) is preferably formed on a pipe connector (321) which defines a cavity (352) at the inner side, wherein the cavity (352) is delimited in the proximal direction by the filter region (350).

5. A discharge device comprising:
a separator element (300) in accordance with any one of the preceding claims; and
a container (100) for receiving a product with a peripheral container wall (110), a proximal container end (101), a distal container end (102) and a discharge opening (104) on the distal container end (102),
wherein an external thread (160) which extends around the axial direction (L) is formed at the container wall (110) in the region of the distal container end (102), and wherein the external thread (160) of the container (100) can be brought into engagement with the internal thread (310) of the separator element (300) in order to attach the separator element (300) to the distal container end (102).

6. A discharge device in accordance with claim 5,
wherein an axial locking structure (170) is formed proximally from the external thread (160) of the container (100) at the outer side on the container wall (110), and
wherein the at least one locking arm (330) of the separator element (300) can be brought into engagement with the axial locking structure (170) such that a proximal movement of the separator element (300) relative to the container (100) is impeded, and can be brought out of engagement by an outward radial deflection of the locking arm (330).

7. A discharge device in accordance with one of the claims 5-6,
wherein a rotational locking structure (190) is formed proximally from the external thread (160) of the container (100) at the outer side on the container wall (110),
wherein a complementary engagement structure (360) is formed at the inner side on at least one locking arm (330) of the separator element (300) and can be brought into engagement with the rotational locking structure (190) such that it impedes a rotation of the separator element (300) relative to the container (100), and can be brought out of engagement by an outward radial deflection of the locking arm (330).

8. A discharge device in accordance with any one of the claims 5-7 comprising a closure (400) with a peripheral outer wall (410), a proximal closure end (401), a distal closure end (402) and a top wall (420), wherein the top wall (420) of the closure (400) axially covers the distal end (302) of the separator element (300) in the closing position, and
wherein the outer wall (410) radially covers the at least one locking arm (330) in the closing position such that the outer wall (410) impedes a radial deflection of the locking arm (330), and
wherein, in the closing position, the outer wall (410) of the closure (400) extends in the proximal direction preferably at least as far as the proximal end (301) of the separator element (300) so that the closure (400) entirely covers the separator element (300) in the closing position.

9. A discharge device in accordance with claim 8,
wherein an internal thread (430) which is open toward the proximal closure end (401) and which extends around the axial direction (L) is formed in the outer wall (410) of the closure (400),
wherein the separator element (300) has an external thread (320) which is open toward the distal end (302) and which extends around the axial direction (L), and
wherein, in the closing position, the external thread (320) of the separator element (300) is in engagement with the internal thread (430) of the closure (400).

10. A discharge device in accordance with claim 9,
wherein the external thread (320) of the separator element (300) and the external thread (160) of the container (100) are of the same dimensions so that the closure (400), with its internal thread (430), can selectively be screwed onto the separator element (300) or directly onto the container (100), and/or
wherein the external thread (320) of the separator element (300) is formed on a pipe connector (321) which defines a cavity (352) at the inner side, wherein the cavity (352) is delimited in the proximal direction by the filter region (350), and
wherein the closure has a plug (421) which is formed at the top wall (420) of the closure (400), wherein the plug (421) extends in the interior of the closure (400) in the direction of the proximal closure end (401) and extends in the closing position into the cavity (352), preferably such that a proximal front face (422) of the plug (421) lies on the filter region (350) in the closing position.

11. A discharge device in accordance with claim 10,
wherein the top wall (450) defines a distal front face (423),
wherein, between the distal front face (423) and the distal closure end (402), the closure (400) has a second internal thread (470) which is open toward the distal closure end (402), which extends around the axial direction (L) and which is formed in a complementary manner to the external thread (160) at the distal container end (102), and
wherein the closure (400) can be screwed with the second internal thread (470) in a reverse orientation relative to the closing position onto the external thread (160) at the distal container end (102) such that the distal front face (423) covers the distal container end (102).

12. A discharge device in accordance with any one of the claims 8-11,
wherein the outer wall (410) of the closure (400) has one or more drivers (440) distally from the top wall (420) at the inner side,
wherein the separator element has one or more driver elements (340) at the outer side, and
wherein the drivers (440) can be brought into engagement with the driver elements (340) of the separator element (300) by the closure (400) being connected to the separator element (300), in particular being pushed onto the separator element (300), in a reverse orientation relative to the closing position along the axial direction (L) so that the separator element (300) can be unscrewed from the container (100) by means of the closure (400).

13. A combination of a discharge device in accordance with any one of the claims 5 to 12 with a packaging (600) which comprises a carrier (600) which defines a horizontal upper side, wherein an upwardly open receiver recess (620) is formed in the carrier (610) for receiving the discharge device in a planar manner,
wherein a closure holding recess (630) is formed in the carrier (610) and is dimensioned in a complementary manner to the closure (400), wherein the closure (400) can be inserted into the closure holding recess (630) such that the distal closure end (401) faces upwardly,
wherein the closure holding recess (630) is formed separately from the receiver recess (620) or is formed as a region of the receiver recess (620).

14. A combination in accordance with claim 13,
wherein the discharge device has a feed element (200) to discharge the product received in the container from the container through the discharge opening after removing the separator element,
wherein the feed element (200) has a thumb ring (230), and wherein the receiver recess (620) has a first recess region (625) for receiving the thumb ring (230), wherein the thumb ring (230) comes to rest in the first recess region (625) when the feed element is located in a first feed position relative to the container (100), and
wherein the receiver recess (620) has a second recess region (626) for receiving the thumb ring (230), wherein the thumb ring (230) comes to rest in the second recess region (625) when the feed element is in a second feed position which differs from the first feed position.

15. A combination in accordance with one of the claims 13-14, wherein an upwardly open separate fluid reservoir recess (640) is formed in the carrier (610) and makes it possible to receive a fluid and to suction this fluid with the discharge device after the closure (400) has been removed from the discharge device.

## Revendications

1. Elément de séparation (300) destiné à un dispositif de distribution médical pour distribuer un produit, ayant une extrémité proximale (301) et une extrémité distale (302), comprenant :
une zone de filtrage (350) réalisée pour empêcher le passage de granulés le long d'une direction axiale (L) entre l'extrémité proximale (301) et
l'extrémité distale (302), mais pour permettre le passage de liquide le long de la direction axiale (L) ; et
un taraudage (310) ouvert vers l'extrémité proximale (301), qui s'étend autour de la direction axiale (L) ;
**caractérisé par** au moins un bras d'enclenchement (330) à élasticité de ressort, disposé sur la périphérie extérieure, qui est réalisé dans la zone de l'extrémité proximale (301) et dont l'extrémité libre s'étend jusqu'à l'extrémité proximale (301),
et en ce que l'élément de séparation (300) comprend un ou plusieurs éléments entraîneurs (340) qui sont réalisés sur le côté extérieur de l'élément de séparation (300) pour permettre un engagement par coopération de forme de l'élément de séparation (300) avec un entraîneur d'un obturateur dans la direction périphérique.

2. Elément de séparation (300) selon la revendication 1,
dans lequel ledit au moins un bras d'enclenchement (330) présente, dans la zone de son extrémité libre, du côté intérieur, un élément de préhension par l'arrière (332), en particulier sous la forme d'un ergot d'enclenchement s'étendant radialement vers l'intérieur, et/ou
ledit au moins un bras d'enclenchement (330) présente, dans la zone de son extrémité libre, du côté intérieur, une structure d'engagement (360) qui est réalisée pour coopérer avec une structure de blocage anti-rotation (190) sur un récipient (100), la structure d'engagement (360) du côté intérieur étant réalisée en particulier sous la forme d'une denture intérieure qui s'étend autour de la direction axiale (L).

3. Elément de séparation (300) selon l'une des revendications précédentes, dans lequel ledit au moins un bras d'enclenchement (330) est réalisé sur la périphérie extérieure de l'élément de séparation (300) de telle sorte qu'une surface extérieure (331) dudit au moins un bras d'enclenchement est disposée à fleur ou décalée radialement vers l'extérieur par rapport à une surface extérieure (311) de l'élément de séparation (300) distale par rapport au bras d'enclenchement, et
de préférence, ledit au moins un bras d'enclenchement (330) s'étend dans la direction axiale (L) jusque dans une zone proximale par rapport au taraudage (310).

4. Elément de séparation (300) selon l'une des revendications précédentes, présentant un filetage (320) ouvert vers l'extrémité distale (302), qui s'étend autour de la direction axiale (L), et
de préférence, le filetage (320) est réalisé sur un embout tubulaire (321) qui définit une cavité (352) du côté intérieur, la cavité (352) étant délimitée dans la direction proximale par la zone de filtrage (350).

5. Dispositif de distribution, comprenant :
un élément de séparation (300) selon l'une des revendications précédentes ; et
un récipient (100) destiné à recevoir un produit, présentant une paroi de récipient périphérique (110), une extrémité de récipient proximale (101), une extrémité de récipient distale (102) et une ouverture de distribution (104) à l'extrémité de récipient distale (102),
dans lequel un filetage (160) est réalisé sur la paroi (110) du récipient dans la zone de l'extrémité distale (102) du récipient, lequel s'étend autour de la direction axiale (L), et le filetage (160) du récipient (100) peut être amené en engagement avec le taraudage (310) de l'élément de séparation (300) afin de monter l'élément de séparation (300) sur l'extrémité distale (102) du récipient.

6. Dispositif de distribution selon la revendication 5,
dans lequel une structure de blocage axial (170) est réalisée sur le côté extérieur de la paroi (110) du récipient, du côté proximal du filetage (160) du récipient (100),
ledit au moins un bras d'enclenchement (330) de l'élément de séparation (300) peut être amené en engagement avec la structure de blocage axial (170) de manière à empêcher un mouvement proximal de l'élément de séparation (300) par rapport au récipient (100), et peut être dégagé par une déviation radiale du bras d'enclenchement (330) vers l'extérieur.

7. Dispositif de distribution selon l'une des revendications 5 à 6,
dans lequel une structure de blocage anti-rotation (190) est réalisée sur le côté extérieur de la paroi (110) du récipient, du côté proximal du filetage (160) du récipient (100),
une structure d'engagement complémentaire (360) est réalisée sur le côté intérieur dudit au moins un bras d'enclenchement (330) de l'élément de séparation (300), qui peut être amenée en engagement avec la structure de blocage anti-rotation (190) de manière à empêcher une rotation de l'élément de séparation (300) par rapport au récipient (100), et qui peut être dégagée par une déviation radiale du bras d'enclenchement (330) vers l'extérieur.

8. Dispositif de distribution selon l'une des revendications 5 à 7, comprenant un obturateur (400) ayant une paroi enveloppe périphérique (410), une extrémité d'obturateur proximale (401), une extrémité d'obturateur distale (402) et une paroi de recouvrement (420), la paroi de recouvrement (420) de l'obturateur (400) recouvrant axialement, dans une position d'obturation, l'extrémité distale (302) de l'élément de séparation (300), et
dans la position d'obturation, la paroi enveloppe (410) recouvrant radialement ledit au moins un bras d'enclenchement (330) de telle sorte que la paroi enveloppe (410) empêche une déviation radiale du bras d'enclenchement (330), et
dans la position d'obturation, la paroi enveloppe (410) de l'obturateur (400) s'étendant en direction proximale de préférence au moins jusqu'à l'extrémité proximale (301) de l'élément de séparation (300), de sorte que dans la position d'obturation, l'obturateur (400) recouvre complètement l'élément de séparation (300).

9. Dispositif de distribution selon la revendication 8,
dans lequel un taraudage (430) ouvert vers l'extrémité proximale (401) de l'obturateur est réalisé dans la paroi enveloppe (410) de l'obturateur (400), lequel s'étend autour de la direction axiale (L),
l'élément de séparation (300) présente un filetage (320) ouvert vers l'extrémité distale (302), lequel s'étend autour de la direction axiale (L), et dans la position d'obturation, le filetage (320) de l'élément de séparation (300) est en engagement avec le taraudage (430) de l'obturateur (400).

10. Dispositif de distribution selon la revendication 9,
dans lequel le filetage (320) de l'élément de séparation (300) et le filetage (160) du récipient (100) sont de même dimension, de sorte que l'obturateur (400) peut être vissé avec son taraudage (430) au choix sur l'élément de séparation (300) ou directement sur le récipient (100), et/ou
le filetage (320) de l'élément de séparation (300) est réalisé sur un embout tubulaire (321) qui définit une cavité (352) du côté intérieur, la cavité (352) étant délimitée dans la direction proximale par la zone de filtrage (350), et l'obturateur comprend un bouchon (421) qui est réalisé sur la paroi de recouvrement (420) de l'obturateur (400), le bouchon (421) s'étendant à l'intérieur de l'obturateur (400) en direction de l'extrémité proximale (401) de l'obturateur et s'étendant jusque dans la cavité (352) dans la position d'obturation, de préférence de telle sorte qu'une surface frontale proximale (422) du bouchon (421) repose sur la zone de filtrage (350) dans la position d'obturation.

11. Dispositif de distribution selon la revendication 10,
dans lequel la paroi de recouvrement (420) définit une face frontale distale (423),
entre la face frontale distale (423) et l'extrémité distale (402) de l'obturateur, l'obturateur (400) présente un deuxième taraudage (470) qui est ouvert vers l'extrémité distale (402) de l'obturateur, s'étend autour de la direction axiale (L) et est complémentaire du filetage (160) à l'extrémité distale (102) du récipient, et
l'obturateur (400) peut être vissé avec le deuxième taraudage (470) sur le filetage (160) à l'extrémité distale (102) du récipient dans une orientation inversée par rapport à la position d'obturation, de sorte que la surface frontale distale (423) recouvre l'extrémité distale (102) du récipient.

12. Dispositif de distribution selon l'une des revendications 8 à 11,
dans lequel la paroi enveloppe (410) de l'obturateur (400) comprend, en direction distale de la paroi de recouvrement (420), du côté intérieur, un ou plusieurs entraîneurs (440),
l'élément de séparation comprend, du côté extérieur, un ou plusieurs éléments entraîneurs (340), et
les entraîneurs (440) peuvent être amenés en engagement avec les éléments entraîneurs (340) de l'élément de séparation (300) du fait que l'obturateur (400) est relié à l'élément de séparation (300), en particulier enfilé sur celui-ci, dans une orientation inversée par rapport à la position d'obturation le long de la direction axiale (L), de sorte que l'élément de séparation (300) peut être dévissé du récipient (100) à l'aide de l'obturateur (400).

13. Combination d'un dispositif de distribution selon l'une des revendications 5 à 12 avec un emballage (600) comprenant un support (610) qui définit une face supérieure horizontale, un renfoncement de réception (620) ouvert vers le haut étant ménagé dans le support (610) pour recevoir le dispositif de distribution en position horizontale,
dans laquelle un renfoncement de retenue d'obturateur (630) est ménagé dans le support (610), lequel est dimensionné de manière complémentaire à l'obturateur (400), l'obturateur (400) pouvant être enfiché dans le renfoncement de retenue d'obturateur (630) de telle sorte que l'extrémité distale (401) de l'obturateur est dirigée vers le haut,
le renfoncement de retenue d'obturateur (630) est réalisé séparément du renfoncement de réception (620) ou est formé comme une zone du renfoncement de réception (620).

14. Combinaison selon la revendication 13,
dans laquelle le dispositif de distribution comprend un élément d'avance (200) pour distribuer le produit, reçu dans le récipient, à travers l'ouverture de distribution hors du récipient, une fois que l'élément de séparation a été retiré,
l'élément d'avance (200) comprend une bague (230) pour le pouce, et
le renfoncement de réception (620) présente une première zone de renfoncement (625) pour recevoir la bague (230) pour le pouce, la bague (230) pour le pouce venant se placer dans la première zone de renfoncement (625) lorsque l'élément d'avance se trouve dans une première position d'avance par rapport au récipient (100), et
le renfoncement de réception (620) présente une deuxième zone de renfoncement (626) destinée à recevoir la bague (230) pour le pouce, la bague (230) pour le pouce venant se placer dans la deuxième zone de renfoncement (625) lorsque l'élément d'avance est dans une deuxième position d'avance différente de la première position d'avance.

15. Combinaison selon l'une des revendications 13 à 14,
dans laquelle un renfoncement formant réservoir de fluide (640) séparé, ouvert vers le haut, est ménagé dans le support (610), qui permet de recevoir un fluide et de l'aspirer au moyen du dispositif de distribution, une fois que l'obturateur (400) a été retiré du dispositif de distribution.
